(19) Europäisches Patentamt
European Patent Office
Office européen des brevets

(11) **EP 2 498 727 B1**

(12) **EUROPÄISCHE PATENTSCHRIFT**

(45) Veröffentlichungstag und Bekanntmachung des Hinweises auf die Patenterteilung:
**22.10.2014 Patentblatt 2014/43**

(21) Anmeldenummer: **10784690.9**

(22) Anmeldetag: **12.11.2010**

(51) Int Cl.:
*A61F 2/64* (2006.01)   *A61F 2/66* (2006.01)
*A61F 2/68* (2006.01)   *A61F 2/76* (2006.01)
*A61F 2/50* (2006.01)

(86) Internationale Anmeldenummer:
**PCT/EP2010/006896**

(87) Internationale Veröffentlichungsnummer:
**WO 2011/057795 (19.05.2011 Gazette 2011/20)**

(54) **VERFAHREN ZUR STEUERUNG EINES ORTHETISCHEN ODER PROSTHETISCHEN GELENKES EINER UNTEREN EXTREMITÄT**

METHOD OF CONTROLLING AN ORTHOTIC OR PROSTHETIC JOINT OF A LOWER EXTREMITY

PROCÉDÉ POUR COMMANDER UNE ARTICULATION ORTHÉTIQUE OU PROTHÉTIQUE AVEC UNE EXTRÉMITÉ INFÉRIEURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2009   DE 102009052887**

(43) Veröffentlichungstag der Anmeldung:
**19.09.2012 Patentblatt 2012/38**

(60) Teilanmeldung:
**14001803.7 / 2 772 232**

(73) Patentinhaber: **Otto Bock Healthcare Products GmbH**
**1070 Wien (AT)**

(72) Erfinder:
• **KAMPAS, Philipp**
**A-1020 Wien (AT)**
• **SEYR, Martin**
**A-1040 Wien (AT)**
• **BOITEN, Herman**
**37085 Göttingen (DE)**
• **KALTENBORN, Sven**
**37115 Duderstadt (DE)**
• **AUBERGER, Roland**
**A-1140 Wien (AT)**

(74) Vertreter: **Stornebel, Kai et al**
**Gramm, Lins & Partner GbR**
**Theodor-Heuss-Strasse 1**
**38122 Braunschweig (DE)**

(56) Entgegenhaltungen:
WO-A1-01/43669      WO-A1-96/41599
DE-A1-102006 021 802   US-A1- 2008 114 272
US-A1- 2008 228 287   US-A1- 2009 030 530

EP 2 498 727 B1

**Beschreibung**

Die Erfindung betrifft ein Verfahren zur Steuerung eines orthetischen oder prothetischen Gelenkes einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei über die Sensoren während der Benutzung des Gelenkes Zustandsinformationen bereitgestellt werden.

Kniegelenke für Orthesen oder Prothesen weisen ein oberes Anschlussteil und ein unteres Anschlussteil auf, die über eine Gelenkeinrichtung miteinander verbunden sind. An dem oberen Anschlussteil sind in der Regel Aufnahmen für einen Oberschenkelstumpf oder eine Oberschenkelschiene angeordnet, während an dem unteren Anschlussteil ein Unterschenkelschaft oder eine Unterschenkelschiene angeordnet ist. Im einfachsten Fall sind das obere Anschlussteil und das untere Anschlussteil durch ein einachsiges Gelenk verschwenkbar miteinander verbunden. Eine solche Anordnung ist nur in Ausnahmefällen ausreichend, um den gewünschten Erfolg, entweder eine Unterstützung bei dem Einsatz einer Orthese oder einem natürlichen Gangbild bei einem Einsatz in einer Prothese, zu gewährleisten.

Um die unterschiedlichen Anforderungen während der verschiedenen Phasen eines Schrittes oder bei anderen Verrichtungen möglichst natürlich darzustellen oder zu unterstützten, sind Widerstandseinrichtungen vorgesehen, die einen Flexionswiderstand und einen Extensionswiderstand bereitstellen. Über den Flexionswiderstand wird eingestellt, wie leicht der Unterschenkelschaft oder die Unterschenkelschiene im Verhältnis zum Oberschenkelschaft oder der Oberschenkelschiene bei einer aufgebrachten Kraft nach hinten schwingt. Der Extensionswiderstand bremst die Vorwärtsbewegung des Unterschenkelschaftes oder der Unterschenkelschiene und bildet unter anderem einen Streckanschlag aus.

Aus dem Stand der Technik, beispielsweise der DE 10 2008 008 284 A1, ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssensor, ein Neigungssensor und/oder ein Kraftsensor. Der Extensionsanschlag wird in Abhängigkeit von den Sensordaten ermittelt.

Die DE 10 2006 021 802 A1 beschreibt eine Steuerung eines passiven Prothesenkniegelenkes mit verstellbarer Dämpfung in Flexionsrichtung zur Anpassung einer Prötheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für ein Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die WO 01/43669 A1 betrifft eine Unterstützungsvorrichtung zum Ersetzen einer Gliedmaße oder einer Funktion einer Gliedmaße mit zumindest zwei Teilen, die über ein künstliches Gelenk miteinander verbunden sind. Ein Sensor ist vorgesehen, der die Position relativ zu einer feststehenden Linie ermittelt, die beispielsweise als Schwerkraftlinie ausgebildet sein kann. Der Sensor ist mit einer Steuerungseinrichtung verbunden, die auf Basis der Sensordaten das Kniegelenk beeinflusst.

Die US 2008/0228287 A1 betrifft ein Prothesenbein mit einem Prothesenkniegelenk, das mit einem Hydraulikzylinder zum Einstellen des Flexions- und Extensionswiderstandes versehen ist. Über mechanische Kopplungseinrichtungen werden Ventile zur Beeinflussung des jeweiligen Widerstandes betätigt.

Die US 2009/0030530 A1 betrifft ein elektronisch gesteuertes Prothesensystem, mit einer Vielzahl von Sensoren und einem magnetoheroischen Fluid, über das die Plantarflexion oder Dorsalflexion eines Prothesenfußes beeinflusst wird. Ein Verfahren sieht eine Steuerung auf der Grundlage der Gehgeschwindigkeit vor, die aufgrund der Winkelgeschwindigkeit und des Absolutwinkels ermittelt wird. Über ein myoelektrisches Sensorsystem kann eine Steuerung erfolgen, bei der auch ein Druck gemessen wird. Als eingebettete Sensoren werden Winkelsensoren, Kraftsensoren, Temperatur-, Druck- und Vibrationssensoren vorgesehen. Kraft- und Drehmomentdaten können aus Winkeln, Winkelgeschwindigkeiten oder Winkelbeschleunigungen errechnet werden.

Die US 2008/114272 A1 betrifft ein Steuerungssystem für ein Prothesenknie, mit der während der Standphase ein Fahrmeter errechnet wird, der sich aus den Eingängen der Sensordaten ergibt. Als Sensoren sind Axialkraftsensoren und Momentensensoren erwähnt.

Die WO 96/41599 A1 betrifft ein Verfahren zur Steuerung einer Kniebremse eines Prothesenkniegelenks. Es werden ein Kniemoment und ein Hüftmoment über Dehnungsmessstreifen ermittelt, indem durch eine Linie auch Extrapolation der beiden Messpunkte der Wert für das Kniemoment und bei weiterer Extrapolation der Wert für das Hüftmoment errechnet wird. Über die ermittelten Momente kann die vorgegebene Gangart beeinflusst werden.

Ziel der Erfindung ist es, ein Verfahren zur Steuerung eines künstlichen Kniegelenkes bereit zu stellen, mit dem eine situationsabhängige Anpassung des Flexionswiderstandes und des Extensionswiderstandes ermöglicht wird. Erfindungsgemäß wird diese Aufgabe durch ein Verfahren mit den Merkmalen des Anspruchs 1 gelöst. Vorteilhafte Ausgestaltungen und Weiterbildungen der Erfindung sind in den Unteransprüchen aufgeführt.

Das erfindungsgemäße Verfahren zur Steuerung eines orthetischen oder prothetischen Gelenkes einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei über die Sensoren während der Benutzung des Kniegelenkes Zustandsinformationen bereitgestellt werden, sieht vor, dass die Sensordaten von zumindest einer Einrichtung zur Erfassung von zumindest

- einem Moment und einer Kraft, oder
- zwei Momenten und einer Kraft, oder
- zwei Kräften und einem Moment

ermittelt und die Sensordaten von zumindest zwei der ermittelten Größen durch eine mathemathematische Operation miteinander verknüpft werden und dadurch zumindest eine Hilfsvariable errechnet wird, die der Steuerung des Beuge- und/oder Streckwiderstandes zugrunde gelegt wird. Die Sensoren, die beispielsweise als Knie- oder Knöchelmomentsensoren oder Axiallastsensoren ausgebildet sein können, stellen Grunddaten zur Verfügung, aus denen eine Hilfsvariable über eine mathematische Operation, beispielsweise Addition, Multiplikation, Subtraktion oder Division, errechnet wird, Diese Hilfsvariable ist ausreichend aussagekräftig, um auf deren Grundlage eine Anpassung der Widerstände zu errechnen, Die Hilfsvariable ermöglicht es auf schnelle Art und Weise ohne hohen Rechenaufwand eine Kenngröße bereitzustellen, über die der aktuelle einzustellende Widerstand als Zielgröße errechnet und der Aktuator entsprechend angesteuert werden kann, um den gewünschten Widerstand zu erreichen. Dabei sind als Hilfsvariable Schnittmomente, Schnittkräfte, Kräfte oder Abstände vorgesehen, wobei als Hilfsvariable beispielsweise Kräfte und Momente ermittelt werden können, die an Stellen der Orthese oder Prothese wirken, die nicht unmittelbar über Sensoren zugänglich sind. Während die Sensoren nur die unmittelbar wirkenden Kräfte oder Momente ermitteln, kann über die Errechnung der Hilfsvariable eine Größe zur Beurteilung der Einstellung der Widerstände herangezogen werden, die nicht unmittelbar erfasst werden muss. Dies erweitert die Möglichkeiten bei der Beurteilung, wann welcher Widerstand in welchem Zustand der Bewegung oder in welcher Stellung des Gelenkes oder der Prothese eingestellt werden sollte. Grundsätzlich ist es möglich, mehrere Hilfsvariablen gleichzeitig zu bestimmen und zur Steuerung einzusetzen.

Als *erfindungsgemäße* Hilfsvariable wird der Abstand eines Kraftvektors zu einer Achse in einer Referenzhöhe, ein Schnittmoment in einer Referenzhöhe oder eine Schnittkraft ermittelt. So kann beispielsweise der Abstand des Bodenreaktionskraftvektors errechnet werden, indem ein Moment durch die Axialkraft dividiert wird. Dazu ist z.B. vorgesehen, dass die zumindest eine Einrichtung zur Erfassung eines Momentes, z.B. ein Momentensensor, ein Kniemoment erfasst, so dass als Hilfsvariable der Abstand des Kraftvektors der Bodenreaktionskraft beispielsweise in Kniehöhe, also in Höhe der Kniegelenkachse, ermittelt wird. Auch ist es möglich, den Abstand zu einer Längsachse zu bestimmen, beispielsweise den Abstand zu einem Referenzpunkt auf einer Längsachse zu bestimmen, wobei die Längsachse die Einrichtungen zur Erfassung der Momente verbindet. So kann beispielsweise der Abstand eines Kraftvektor zu der Längsachse des unteren Anschlussteils an dem Kniegelenk, also dem Unterschenkelteil, verwendet werden. Als Hilfsvariable *wird* der Abstand des Kraftvektor zu einer Achse eines Gelenkanschlussteiles in einer Referenzposition durch Verknüpfung der Daten von zumindest einer Einrichtung zur Erfassung zweier Momente und einer Kraft ermittelt.

Die Sensoren sind beispielsweise an dem Unterschenkelschaft oder der Unterschenkelschiene sowie im Bereich der Gelenke angeordnet. Die Hilfsvariable kann eine physikalische Größe in Gestalt eines virtuellen Sensors darstellen. Da sie unter anderem aus Momenten, Kräften und geometrischen Abmessungen des künstlichen Gelenkes errechnet wird, kann als Hilfsvariable *neben dem Abstand einen Kraft von einer Bezugspunkt oder einer Referenzhöhe* eine Kraft, ein Schnittmoment oder eine Schnittkraft in einer Referenzhöhe ermittelt werden.

Grundsätzlich ist es auch möglich, andere Referenzhöhen zu verwenden, indem die Einrichtung zur Erfassung eines Momentes auf Höhe der Referenzhöhe angebracht ist oder indem das Moment auf einer Referenzhöhe durch gewichtete Addition von zwei Momenten, die sich nicht auf der Referenzhöhe befinden, errechnet wird. Es können als *weitere* Hilfsvariable ein Schnittmoment oder eine Schnittkraft durch eine Komponente in einer Referenzhöhe ermittelt werden. Die Hilfsvariable, die mit dem virtuellen Sensor, also durch mathematische Verknüpfung mehrerer Sensorseite, erfasst wird, wird in einer Recheneinheit, beispielsweise in einem Mikroprozessor, errechnet.

Im Speziellen können folgende Größen als Hilfsvariablen zur Steuerung eines künstlichen Kniegelenkes hervorgehoben werden, nämlich der Abstand der Bodenreaktionskraft zu der Kniegelenkachse oder das Moment der Bodenreaktionskraft um die Knieachse, der Abstand der Bodenreaktionskraft in Höhe des Fußes oder das Moment, das die Bodenreaktionskraft um die Unterschenkelachse auf der Höhe des Fußes, insbesondere auf Höhe des Fußbodens erzeugt.

Eine weitere Möglichkeit zu Verrechnung der *erfindungsgemäßen* Hilfsvariable besteht darin, dass der Abstand des Kraftvektors zur Unterschenkelachse in einer Referenzposition durch die Verknüpfung von Daten zweier Einrichtungen zur Erfassung eines Momentes und eines Axialkraftsensors ermittelt wird. Wenn von einem Momentensensor gesprochen wird, umfasst diese Formulierung auch Einrichtungen zur Erfassung eines Momentes, die sich aus mehreren Komponenten zusammensetzen und nicht notwendigerweise an dem Ort angeordnet sein müssen, an dem das Moment wirkt.

Auch ist es möglich, dass ein Schnittmoment in einer Referenzhöhe, durch eine gewichtete Addition oder Subtraktion der Werte eines Knöchelmomentsensors und eines Kniemomentsensors ermittelt wird. Das Schnittmoment ist dann *eine weitere* Hilfsvariable, auf deren Grundlage die Steuerung entsprechend eingestellt wird.

Weiterhin ist es möglich und vorgesehen, dass als *weitere* Hilfsvariable eine auf ein unteres Anschlussteil, z. B. den Fuß, ausgeübte Querkraft aus dem Quotienten der Differenz zweier Momente, z.B. von Kniemoment und Knöchelmoment, und dem Abstand der Momentsensoren ermittelt wird. Auf der Grundlage der ermittelten Hilfsvariable oder mehrerer Hilfsvariablen wird dann der entsprechende Widerstandswert errechnet und eingestellt. Nach Überschreiten des Maximums für die Hilfsvariable kann der Widerstand kontinuierlich mit der Hilfsvariable gesenkt werden, um ein leichteres Durchschwingen des Gelenks auf Rampen oder Treppen zu ermöglichen.

Bei Erreichen oder Überschreiten eines vorbestimmten Wertes für die Hilfsvariable kann die Widerstandseinrichtung in den Schwungphasenzustand geschaltet werden, wodurch sich eine gegenüber dem Standphasenzustand geänderte Grundeinstellung der Flexionsdämpfung und Extensionsdämpfung ergibt. Hierfür bietet sich das Schnittmoment oder der Abstand des Bodenreaktionskraftvektors auf Höhe des Fußes an.

Es ist vorgesehen, dass Sensoren zur Ermittlung des Kniewinkels, einer Kniewinkelgeschwindigkeit, einer Oberschenkelschienenposition oder einer Oberschenkelschaftposition, einer Unterschenkelposition oder Unterschenkelschaftposition, der Änderung dieser Positionen und/oder der Beschleunigung der Orthese oder Prothese vorhanden sind und dass auch deren Daten, neben der Verwendung der Hilfsvariable, zur Steuerung des Widerstandes bzw. der Widerstände verwendet werden.

Damit eine möglichst reibungslose Anpassung des Widerstandes an die Zustandsbedingungen erfolgt, ist vorgesehen, dass sowohl die Datenerfassung als auch die Berechnung der Hilfsvariable und die Widerstandsanpassung in Echtzeit erfolgen. Bevorzugt erfolgt die Widerstandsveränderung kontinuierlich mit der Hilfsvariable und/oder den Sensordaten, um eine sanfte Anpassung der Steuerungsänderung vorzunehmen, so dass der Orthesenoder Prothesennutzer nicht mit abrupten Änderungen des Verhaltens der Orthese oder Prothese konfrontiert wird.

Weiterhin ist vorgesehen, dass bei einer festgestellten Entlastung, also Reduzierung der Bodenreaktionskraft auf die Orthese oder Prothese, beispielsweise beim Anheben des Beines, der Flexionswiderstand verringert und bei einer zunehmenden Belastung der Flexionswiderstand erhöht wird. Bei einer solchen Stehfunktion, die latent vorhanden und immer ausgeführt wird, wenn das natürliche Bewegungsmuster eintritt, kann der Widerstand bis zu einer Sperrung des Gelenkes führen. Die Erhöhung und Verringerung des Widerstandes erfolgt bevorzugt kontinuierlich und ermöglicht einen sanften Übergang, was der natürlichen Bewegung angenähert ist und zu einem sicheren Gefühl bei dem Prothesenoder Orthesenträger führt. Ändert sich die Hilfsvariable, kann die Sperre oder die Erhöhung des Widerstandes, die in der Stehfunktion aktiviert wurde, aufgehoben oder verringert werden, beispielsweise aufgrund der Veränderung der Lage der Prothese oder Orthese im Raum.

Grundsätzlich ist es vorgesehen, dass der Übergang von der Standphase in die Schwungphase belastungsabhängig erfolgt, ebenfalls ist es möglich, aus der Widerstandseinstellung für die Standphase in die Widerstandseinstellung für die Schwungphase durch eine allmähliche Anpassung der Widerstände hineinzugleiten und bei Bedarf, also bei Vorliegen entsprechender Daten für die Hilfsvariable, ebenso allmählich wieder in die Standphase zurückzukehren. Dies ist vorteilhaft, insbesondere um eine Schwungphase auf der Rampe zu ermöglichen, indem als Hilfsvariable die Querkraft im Unterschenkel verwendet wird.

Ein weiterer Aspekt der Erfindung sieht vor, dass der Widerstand in Abhängigkeit einer gemessenen Temperatur verändert wird. Dadurch ist es möglich, die Widerstandseinrichtung oder auch andere Komponenten des künstlichen orthetischen oder prothetischen Gelenkes vor einer zu starken Erwärmung zu schützen. Eine Erwärmung kann soweit führen, dass das Gelenk versagt, weil Teile des Gelenkes die Form oder die strukturelle Festigkeit verlieren oder weil die Elektronik außerhalb der erlaubten Betriebsparameter betrieben wird. Der Widerstand wird dabei bevorzugt so verändert, dass die dissipierte Energie verringert wird. Aufgrund der geringeren umzuwandelnden Energiemenge können sich die Widerstandseinrichtung oder andere Komponenten des künstlichen Gelenkes abkühlen und in einem Temperaturbereich arbeiten, für den sie vorgesehen sind. Darüber hinaus kann vorgesehen sein, dass die Widerstandseinrichtung so angepasst wird, dass Veränderungen, die aufgrund einer Temperaturveränderung auftreten, ausgeglichen werden. Verringert sich beispielsweise die Viskosität einer Hydraulikflüssigkeit aufgrund der Erwärmung, kann die Widerstandseinrichtung entsprechend verstellt werden, um weiterhin die gewohnten Flexionswiderstände und Extensionswiderstände bereitzustellen, damit der Prothesen- oder Orthesennutzer weiterhin auf ein ihm bekanntes Verhalten des künstlichen Gelenkes vertrauen kann.

In einer Variante ist vorgesehen, dass für die Standphase, z.B. während des Gehens, bei zunehmender Temperatur der Widerstand erhöht wird. Dabei kann sowohl der Extensionsals auch der Flexionswiderstand erhöht werden. Durch den erhöhten Widerstand ist der Nutzer gezwungen, langsamer zu gehen und kann somit weniger Energie in das Gelenk einbringen. Dadurch kann das Gelenk abkühlen, so dass es innerhalb der zulässigen Betriebsparameter betrieben werden kann.

Eine weitere Variante sieht vor, dass beim Gehen für die Schwungphase der Beugewiderstand bei zunehmender Temperatur verringert wird. Wird in der oder für die Schwungphase der Beugewiderstand reduziert, führt dies dazu, dass das Gelenk weiter ausschwingt. Der Prothesenfuß gelangt damit später nach vorne zum Fersenauftritt, wodurch der Nutzer wiederum gezwungen wird, langsamer zu gehen, was zu einer reduzierten Energieumwandlung in Wärme führt.

Der Widerstand kann bei Erreichen oder bei Überschreiten eines Temperaturschwellwertes verändert werden. Der Widerstand kann dabei sprungartig bei Erreichen oder Überschreiten einer Temperaturschwelle verändert werden, so dass ein Umschalten des Widerstandswertes bzw. der Widerstandswerte erfolgt. Vorteilhaft ist vorgesehen, dass eine kontinuierliche Veränderung des Widerstandes mit der Temperatur erfolgt, nachdem der Temperaturschwellwert erreicht wird. Wie hoch der Temperaturschwellwert angesetzt wird, liegt an den jeweiligen konstruktiven Parametern, verwendeten Materialien und der angestrebten Gleichmäßigkeit des Widerstandsverhaltens der Prothese oder Orthese. Der Widerstand in der Standphase darf unter anderem nicht so weit erhöht werden, dass dadurch eine sicherheitskritische Situation, z.B. beim Treppabsteigen, erzeugt wird.

Die temperaturinduzierte Widerstandsänderung ist nicht der einzige Steuerungsparameter einer Widerstandsänderung, vielmehr ist vorgesehen, dass eine solche temperaturinduzierte Widerstandsänderung einer funktionalen Widerstandsänderung überlagert wird. Ein künstliches Gelenk, beispielsweise ein Kniegelenk oder ein Knöchelgelenk, wird über eine Vielzahl von Parametern situationsabhängig gesteuert, so dass so genannte funktionale Widerstandsänderungen, die beispielsweise auf der Grundlage der Gehgeschwindigkeit, der Gehsituation oder dergleichen erfolgen, durch die Widerstandsänderung aufgrund der Temperatur ergänzt werden.

Es kann weiterhin vorgesehen sein, dass bei Erreichen oder Überschreiten eines Temperaturschwellwertes ein Warnsignal ausgegeben wird, um dem Nutzer der Prothese oder Orthese bewusst zu machen, dass sich das Gelenk oder die Widerstandseinrichtung in einem kritischen Temperaturbereich befindet. Das Warnsignal kann als ein taktiles, optisches oder akustischen Warnsignal ausgegeben werden. Ebenfalls sind Kombinationen der verschiedenen Ausgabemöglichkeiten vorgesehen.

Bevorzugt wird die Temperatur der Widerstandseinrichtung gemessen und der Steuerung zugrunde gelegt, alternativ dazu können auch andere Einrichtungen der Temperaturmessung unterzogen werden, wenn diese ein temperaturkritisches Verhalten aufweisen. Ist beispielsweise eine Steuerungselektronik besonders temperaturempfindlich, empfiehlt es sich, diese alternativ oder ergänzend zu der Widerstandseinrichtung zu überwachen und einen entsprechenden Temperatursensor dort vorzusehen. Sind einzelne Komponenten, beispielsweise aufgrund der verwendeten Materialien, temperaturempfindlich, empfiehlt es sich, an den entsprechenden Stellen eine Messeinrichtung vorzusehen, um entsprechende Temperatursignale erhalten zu können.

Es kann eine Einstellvorrichtung vorgesehen sein, über die der Grad der Widerstandsänderung verändert wird. Beispielsweise kann auf der Grundlage ermittelter Daten, beispielsweise des Gewichtes des Nutzers der Orthese oder Prothese oder der ermittelten Axialkraft beim Auftreten, erkannt werden, dass eine überproportional hohe Widerstandsänderung erfolgen muss. Ebenfalls besteht die Möglichkeit, dass eine manuelle Einstellvorrichtung vorgesehen ist, über die eine Anpassung der jeweiligen Widerstandsänderung erfolgt, so dass eine tendenziell größere oder geringere Widerstandsänderung in Abhängigkeit von eingestellten oder ermittelten Daten erfolgen kann.

Eine Vorrichtung zur Durchführung des Verfahrens, wie es oben beschrieben ist, sieht vor, dass eine einstellbare Widerstandseinrichtung, die zwischen zwei gelenkig aneinander angeordneten Komponenten eines künstlichen orthetischen oder prothetischen Gelenkes angeordnet ist, sowie mit einer Steuereinrichtung und Sensoren, die Zustandsinformationen in der Vorrichtung erfassen, vorhanden sind. Es ist eine Einstellvorrichtung vorgesehen, über die eine Widerstandsänderung aktivierbar und/oder deaktivierbar ist. Dadurch ist es möglich, z.B. eine wahlweise temperaturgesteuerte Widerstandsänderung vorzunehmen und besondere Modi, Funktion oder Zusatzfunktion z.B. eines Kniesteuerungsverfahrens bewusst zu aktivieren oder zu deaktivieren.

Eine Weiterbildung der Erfindung sieht vor, dass der Beuge- und/oder Streckwiderstand während der Schwung- und/oder Standphase oder während des Stehens auf der Grundlage von Sensordaten angepasst wird. Während aus dem Stand der Technik bekannt ist, nach dem einmaligen Erreichen eines Einstellungswertes für die Schwung- oder Standphase diesen beizubehalten, bis eine neue Gangphase eintritt, ist erfindungsgemäß vorgesehen, dass eine Anpassung des Flexions- und/oder Extensionswiderstandes während der Stand-und/oder Schwungphase variabel eingestellt wird. Während der Standphase oder der Schwungphase erfolgt also eine kontinuierliche Anpassung des Widerstandes bei sich verändernden Zuständen, beispielsweise sich erhöhenden Kräften, Beschleunigungen oder Momenten. Statt des Einstellens von Flexionswiderstand und Extensionswiderstand über Schaltschwellen, die nach dem einmaligen Erreichen die Grundlage für die Einstellung der jeweiligen Widerstände bilden, wird erfindungsgemäß vorgesehen, dass eine variable, angepasste Einstellung der Widerstände erfolgt, beispielsweise auf der Basis einer Auswertung von Kennfeldern. Es ist vorgesehen, dass ein Kennfeld für den Flexionswiderstand über dem Kniehebel und dem Kniewinkel aufgestellt wird und die Steuerung des Widerstandes auf der Grundlage des Kennfeldes erfolgt.

Um künstliche Gelenke auf der Grundlage von Sensordaten zu steuern, werden diejenigen Sensoren angeordnet, die gerade notwendig sind, um einen Sicherheitsstandard bei der Detektierung von Gangphasenübergängen zu gewährleisten. Werden über das Mindestmaß hinausgehende Sensoren verwendet, z.B. um den Sicherheitsstandard zu erhöhen, ermöglicht diese Redundanz von Sensoren, Steuerungen zu realisieren, die nicht alle in oder an dem Gelenk angeordneten Sensoren nutzen und dennoch einen Mindeststandard an Sicherheit einhalten. Es ist vorgesehen, dass die Redundanz der Sensoren genutzt wird, um alternative Steuerungen zu realisieren, die im Falle eines Ausfalles von Sensoren mit den noch arbeitenden Sensoren immer noch ein Gehen mit Schwungphase ermöglichen und einen Mindeststandard an Sicherheit bieten.

Weiterhin kann vorgesehen sein, dass der Abstand des Bodenreaktionskraftvektors zu einem Gelenkteil ermittelt wird und der Widerstand dann reduziert wird, wenn ein Schwellwert des Abstandes überschritten wird, also wenn der Abstand des Bodenreaktionskraftvektors über einen Mindestabstand zu einem Gelenkteil liegt, beispielsweise zu einem Punkt auf der Längsachse des Unterschenkelteils in einer bestimmten Höhe oder zu der Schwenkachse des Kniegelenkes.

Der Flexionswiderstand kann in der Standphase auf einen für die Schwungphase geeigneten Wert reduziert werden, wenn unter anderem ein relativ zur Vertikalen hin zunehmender Inertialwinkel des Unterschenkelteils ermittelt wird. Der zunehmende Inertialwinkel des Unterschenkelteils zeigt an, dass sich der Prothesennutzer oder Orthesennutzer in einer Vorwärtsbewegung befindet, wobei als Angelpunkt das distale Ende des Unterschenkelteils angenommen wird. Es ist vorgesehen, dass die Reduzierung nur dann erfolgt, wenn die Zunahme des Inertialwinkels über einem Schwellwert liegt. Weiterhin kann der Widerstand reduziert werden, wenn die Bewegung des Unterschenkelteils relativ zu dem Oberschenkelteil nicht beugend, also streckend ist oder konstant bleibt, was auf eine Vorwärtsbewegung deutet. Gleichfalls kann der Widerstand reduziert werden, wenn ein streckendes Kniemoment vorliegt.

Es kann vorgesehen sein, dass der Widerstand in der Standphase nur reduziert wird, wenn der Kniewinkel weniger als 5° beträgt. Dadurch wird ausgeschlossen, dass während der Schwungphase und bei einem gebeugten Knie das Gelenk ungewünscht freischaltet.

Der Widerstand kann auch bei beugendem Kniemoment auf einen für die Schwungphase geeigneten Wert reduziert werden, wenn ermittelt wurde, dass sich das Kniemoment von streckend auf beugend verändert hat. Die Reduzierung erfolgt dabei unmittelbar nach der Veränderung des Kniemomentes von streckend auf beugend.

Weiterhin kann vorgesehen sein, dass der Widerstand nach einer Reduzierung wieder auf den Wert in der Standphase erhöht wird, wenn innerhalb einer festgelegten Zeit nach der Reduzierung des Widerstandes ein Schwellwert für einen Inertialwinkel einer Gelenkkomponente, für eine Inertialwinkelgeschwindigkeit, für eine Bodenreaktionskraft, für ein Gelenkmoment, für einen Gelenkwinkel oder für einen Abstand eines Kraftvektors zu einer Gelenkkomponente nicht erreicht wird. Anders ausgedrückt, das Gelenk wird wieder in den Standphasenzustand eingestellt, sollte nicht innerhalb einer festgelegten Zeit nach einem Wechsel in den Schwungphasenzustand tatsächlich eine Schwungphase festgestellt werden. Dies beruht darauf, dass die Schwungphasenauslösung bereits stattfindet, bevor die Fußspitze den Boden verlassen hat, um eine rechtzeitige Schwungphaseneinleitung zu ermöglichen. Sollte die Schwungphase dann aber nicht eingeleitet werden, wie zum Beispiel bei einer Zirkumduktionsbewegung der Fall ist, muss wieder auf den sichernden Standphasenwiderstand geschaltet werden. Hierzu ist ein Timer vorgesehen, der überprüft, ob innerhalb einer bestimmten Zeit ein erwarteter Wert für eine der oben bezeichneten Größen vorliegt. Der Widerstand bleibt reduziert, also die Schwungphase bleibt aktiviert, wenn eine Gelenkwinkelzunahme detektiert wird, also wenn tatsächlich eine Schwungphase eingeleitet wird. Ebenfalls ist es möglich, dass nach dem Erreichen des Schwellwertes und dem Freischalten der Schwungphase der Timer erst dann eingeschaltet wird, wenn ein zweiter Schwellwert, der kleiner als der erste Schwellwert ist, unterschritten wird.

Die Erfindung kann weiterhin vorsehen, dass der Beugewiderstand in der Standphase erhöht oder nicht verringert wird, wenn ein in Richtung auf die Vertikale abnehmender Inertialwinkel eines Unterschenkelteils und eine Vorfußbelastung ermittelt wird. Durch die Koppelung der Sensorgröße eines abnehmenden Inertialwinkels eines Unterschenkelteils in Richtung auf die Vertikale und das Vorhandensein einer Vorfußbelastung ist es möglich, das Rückwärtsgehen sicher zu detektieren und keine Schwungphase auszulösen, also den Flexionswiderstand nicht zu verringern, um eine ungewollte Beugung des Kniegelenkes zu vermeiden, wenn beim Rückwärtsgehen das versorgte Bein nach hinten gestellt und aufgesetzt wird. Dadurch ist es möglich, dass das versorgte Bein in Beugerichtung belastet wird, ohne einzuknicken, so dass es einem mit einer Prothese oder Orthese versorgten Patienten möglich ist, ohne eine gesonderte Verriegelung aktivieren zu müssen, rückwärts zu gehen.

Eine Weiterbildung der Erfindung sieht vor, dass der Widerstand erhöht oder zumindest nicht verringert wird, wenn die Inertialwinkelgeschwindigkeit eines Gelenkteils einen Schwellwert unterschreitet oder anders ausgedrückt, dass eine Schwungphase mit einer Absenkung des Flexionswiderstandes eingeleitet wird, wenn die Inertialwinkelgeschwindigkeit einen vorbestimmten Schwellenwert überschreitet. Ebenfalls ist es möglich, dass über die Bestimmung des Inertialwinkels eines Gelenkteils, insbesondere des Unterschenkelteils und der Inertialwinkelgeschwindigkeit eines Gelenkteils, insbesondere des Unterschenkelteils, ermittelt wird, dass sich der Prothesennutzer oder Orthesennutzer rückwärts bewegt und ein gegen eine Flexion verriegeltes oder hoch gebremstes Kniegelenk benötigt. Dementsprechend wird der Widerstand, sofern er noch nicht ausreichend hoch ist, erhöht.

Weiterhin kann vorgesehen sein, dass der Verlauf der Vorfußbelastung ermittelt wird und der Widerstand erhöht oder nicht verringert wird, wenn bei einem abnehmenden Inertialwinkel des Unterschenkelteils sich die Vorfußbelastung verringert. Während bei einer Vorwärtsbewegung nach dem Fersenstoß sich die Vorfußbelastung erst dann erhöht, wenn das Unterschenkelteil über die Vertikale hinaus nach vorne verschwenkt wurde, nimmt die Vorfußbelastung beim Rückwärtsgehen bei einem abnehmenden Inertialwinkel ab, so dass bei Vorliegen der beiden Zustände, nämlich einem abnehmenden Inertialwinkel und einer abnehmenden Vorfußbelastung auf ein Rückwärtsgehen geschlossen werden kann. Dementsprechend wird dann der Widerstand auf denjenigen Wert erhöht, der zum Rückwärtsgehen vorgesehen ist.

Eine weitere Kenngröße kann das Kniemoment sein, das erfasst wird und als Grundlage dafür dient, ob der Widerstand erhöht oder nicht verringert wird. Wenn ein in Flexionsrichtung wirkendes Kniemoment ermittelt wird, also wenn der Prothesenfuß aufgesetzt wurde und ein Flexionsmoment im Knie detektiert wird, liegt eine Situation vor, bei der ein Rückwärtsgehen anzunehmen ist, so dass dann eine Flexionssperre, also ein Erhöhen des Widerstandes auf einen Wert, der ein Beugen ohne weiteres nicht ermöglicht, gerechtfertigt ist.

Es kann weiterhin vorgesehen sein, dass der der Kraftangriffspunkt an dem Fuß bestimmt wird und der Widerstand erhöht oder nicht verringert wird, wenn sich der Kraftangriffspunkt in Richtung Ferse bewegt

Der Inertialwinkel des Unterschenkelteils kann direkt über eine Sensoreinrichtung, die an dem Unterschenkelteil angeordnet ist, bestimmt werden oder aus dem Inertialwinkel eines anderen Anschlussteils, beispielsweise des Oberschenkelteils und einem gleichfalls ermittelten Gelenkwinkel. Da der Gelenkwinkel zwischen dem Oberschenkelteil und dem Unterschenkelteil auch für andere Steuerungssignale eingesetzt werden kann, ist durch die Mehrfachanordung von Sensoren und die mehrfache Nutzung der Signale eine Redundanz gegeben, so dass auch bei Ausfall eines Sensors die Funktionalität der Prothese oder Orthese weiterhin erhalten bleibt. Eine Veränderung des Inertialwinkels eines Gelenkteiles kann direkt über ein Gyroskop oder aus der Differenzierung eines Inertialwinkelsignals des Gelenkteiles oder aus dem Inertialwinkelsignal eines Anschlussteiles und einem Gelenkwinkel ermittelt werden.

Nachfolgend wird ein Ausführungsbeispiel der Erfindung näher beschrieben.

Es zeigen:

Figur 1      eine schematische Darstellung einer Prothese;
Figur 2      eine schematische Darstellung zur Berechnung eines Abstandes;
Figur 3      einen schematische Darstellung zur Berechnung eines Schnittmomentes;
Figur 4      eine schematische Darstellung zur Berechnung eines Abstandes auf der Grundlage mehrerer Sensorwerte;
Figur 5      eine schematische Darstellung zur Berechnung einer Querkraft;
Figur 6      Darstellungen von Werteverläufen des Kniewinkels und einer Hilfsvariable über die Zeit;
Figur 7 -    das Verhalten von Kenngrößen bei zunehmendem Widerstand in der Standphase;
Figur 8 -    das Verhalten von Kenngrößen bei zunehmendem Widerstand in der Schwungphase;
Figur 9 -    einen Kniewinkelverlauf und eine Widerstandskurve beim Gehen in der Ebene;
Figur 10 -   einen Kniewinkelverlauf und eine Widerstandskurve beim Gehen auf einer schiefen Ebene
Figur 11 -   eine Darstellung der Vorzeichenkonvention für den Inertialwinkel und schematische Darstellung einer Prothese während des Rückwärtsgehens;
Figur 12 -   eine Darstellung der Vorzeichenkonvention für Kniewinkel und Kniemoment
Figur 13 -   ein Kennfeld für den Widerstand über dem Kniewinkel und dem Kniehebel;
Figur 14 -   Kenngrößen beim Gehen auf schrägen Ebenen; sowie
Figur 15 -   das Widerstandsverhalten bei unterschiedlichen Querkraftmaxima.

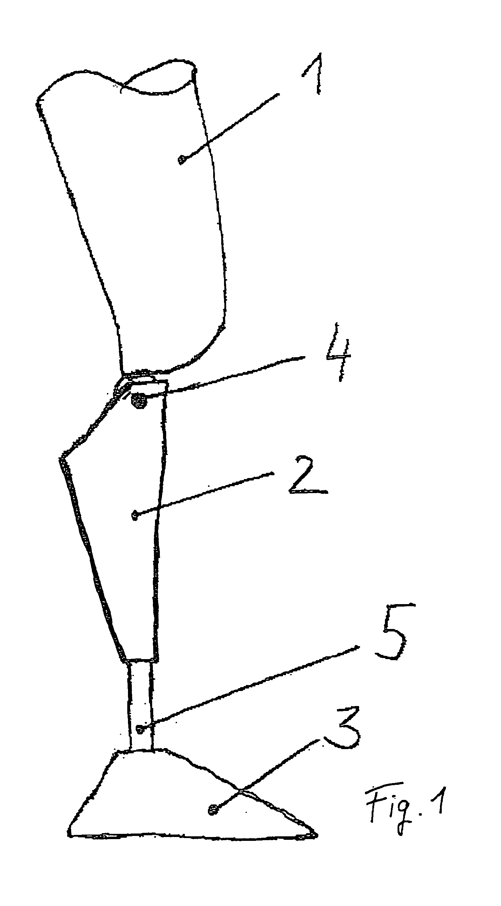

In der Figur 1 ist in einer schematischen Darstellung einer Beinprothese mit einem Oberschenkelschaft 1 zur Aufnahme eines Oberschenkelstumpfes gezeigt. Der Oberschenkelschaft 1 wird auch als oberes Anschlussteil bezeichnet. An dem oberen Anschlussteil 1 ist ein unteres Anschlussteil 2 in Gestalt eines Unterschenkelschaftes mit einer Widerstandseinrichtung angeordnet. An dem unteren Anschlussteil 2 ist ein Prothesenfuß 3 angeordnet. Das untere Anschlussteil 2 ist an dem oberen Anschlussteil 1 über ein Gelenk 4 schwenkbar befestigt. In dem Gelenk 4 ist ein Momentensensor angeordnet, der das wirksame Kniemoment ermittelt. In dem unteren Anschlussteil 2 ist ein Verbindungsteil 5 zu dem Prothesenfuß 3 vorgesehen, in dem eine Einrichtung zur Ermittlung der wirksamen Axialkraft sowie des Knöchelmomentes untergebracht ist. Es können auch Winkelsensoren und/oder Beschleunigungsmesser vorhanden sein. Es ist möglich, dass nicht alle Sensoren in einer Beinprothese oder zusätzliche Sensoren vorhanden sind.

In dem unteren Anschlussteil 2 befindet sich neben der Widerstandseinrichtung, die den Beuge- und Streckwiderstand bereitstellt, eine Steuereinrichtung, über die es möglich ist, den jeweiligen Widerstand auf der Grundlage der empfangenen Sensordaten und der Auswertung der Sensordaten zu verändern. Dazu ist es vorgesehen, dass die Sensordaten zur Erzeugung zumindest einer Hilfsvariable verwendet werden, die über eine mathematische Verknüpfung zweier oder mehrerer Sensordaten erhalten wird. Dadurch ist es möglich, dass mehrere Kraft- oder Momentensensoren miteinander verknüpft werden, um Kräfte, Abstände und/oder Momente zu errechnen, die nicht unmittelbar im Bereich der Sensoren herrschen. So ist es beispielsweise möglich, Schnittkräfte, Schnittmomente oder Abstände in bestimmten Referenzebenen zu errechnen, um ausgehend davon beurteilen zu können, welche Funktionen gegenwärtig ausgeführt werden soll, damit ein möglichst natürliches Gangbild erreicht werden kann. Als Funktion werden dabei diejenigen Steuerungsabläufe bezeichnet, die im Rahmen einer natürlichen Bewegung auftreten, ein Modus hingegen ist ein Schaltungszustand, der durch einen willkürlichen Akt eingestellt wird, beispielsweise durch Betätigung eines gesonderten Schalters oder durch eine bewusste, ggf. bewusst unnatürliche Abfolge von Bewegungen.

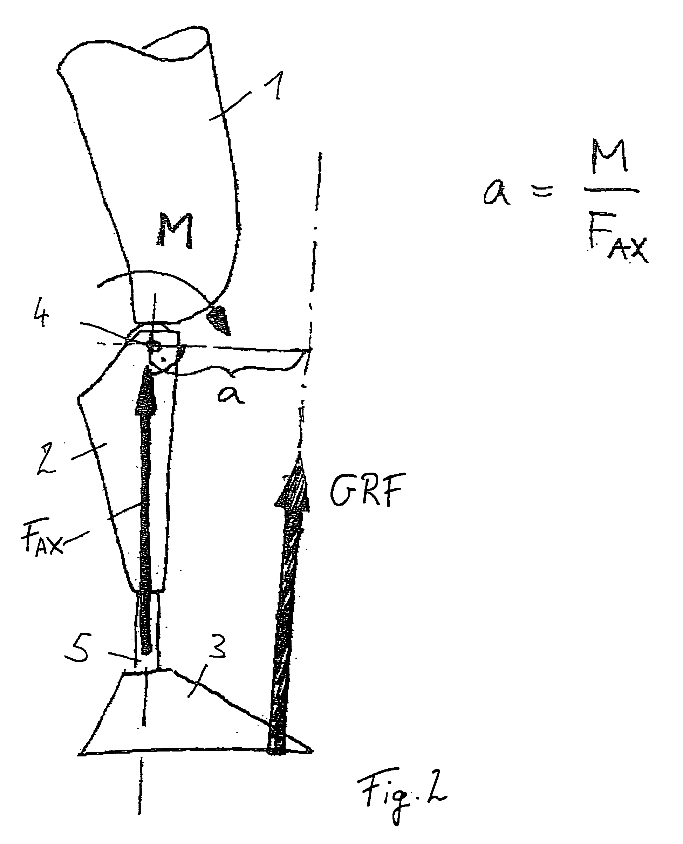

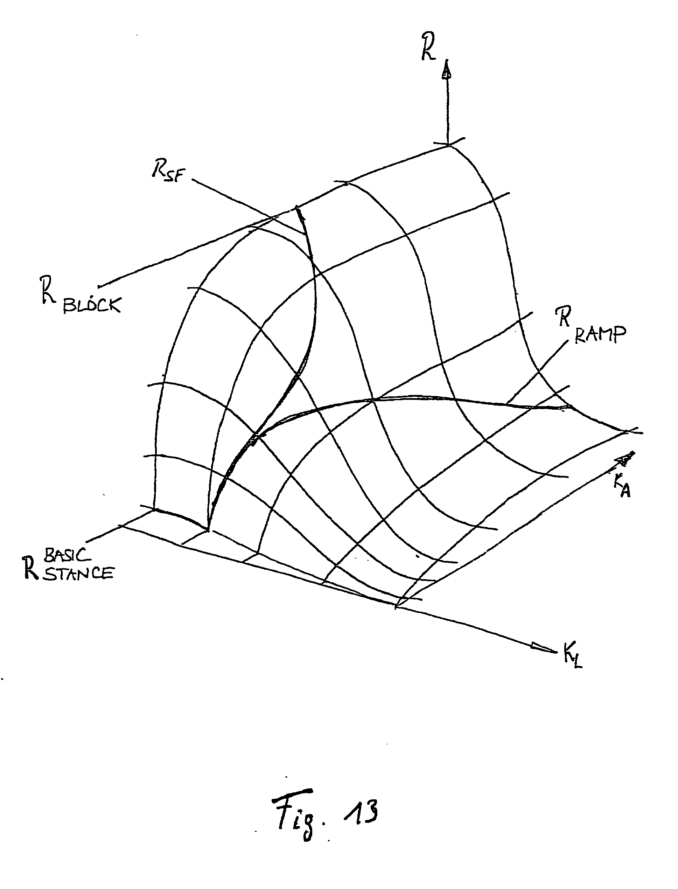

In der Figur 2 ist schematisch dargestellt, wie als Hilfsvariable der Abstand a des Abstandes des Bodenreaktionskraftvektors GRF zu dem Momentensensor berechnet wird. Die Hilfsvariable a ist im vorliegenden Fall der sogenannte Kniehebel, der in Figur 13 ebenfalls dargestellt ist und im Zusammenhang mit einer Kennfeldsteuerung -dort allerdings mit umgekehrtem Vorzeichen- beschrieben werden wird. Der Abstand a errechnet sich aus dem Quotienten des Kniemomentes M und der Axialkraft $F_{AX}$. Je größer das Kniemoment M im Verhältnis zur Axialkraft $F_{AX}$ ist, desto größer ist der Abstand a des Bodenreaktionskraftvektors GRF in der Referenzhöhe, die vorliegend die Knieachse bildet. Auf der Grundlage der Hilfsvariable a ist es möglich, den Streckwiderstand und/oder den Beugewiderstand zu variieren, da über diese Hilfsvariable a errechnet werden kann, ob und in welchem Stadium der Standphase oder Schwungphase sich die Prothese befindet, sodass ausgehend davon ein vorher festgelegter Beuge- und/oder Streckwiderstand eingestellt wird. Durch die Veränderung der Hilfsvariable a kann bestimmt werden, wie die gegenwärtige Bewegung verläuft, sodass innerhalb der Bewegung, auch innerhalb der Standphase oder der Schwungphase, eine Anpassung des Streckund/oder Beugewiderstandes erfolgen kann. Die Veränderung der Widerstände erfolgt bevorzugt kontinuierlich und in Abhängigkeit von der Veränderung der Hilfsvariable oder der Hilfsvariablen.

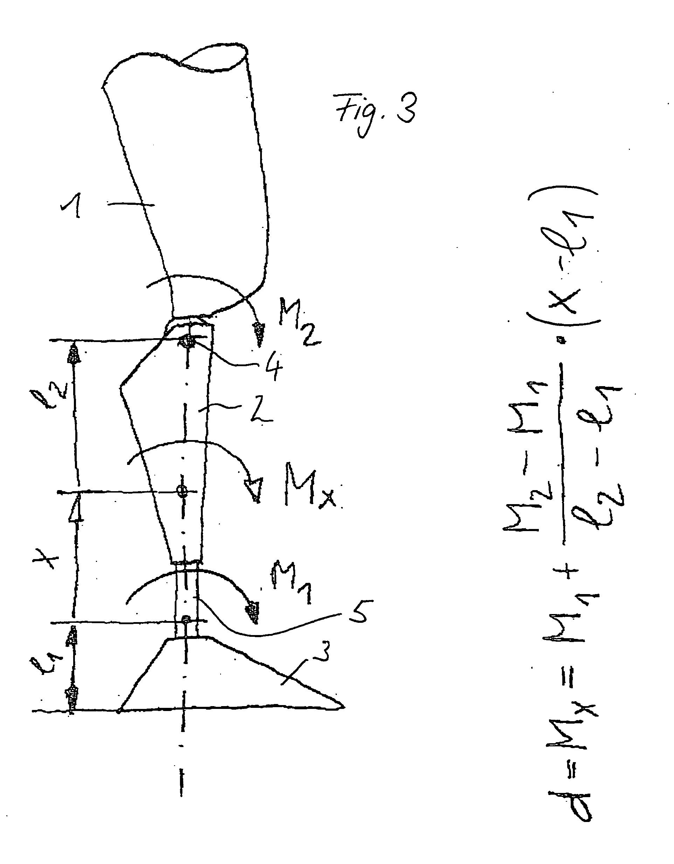

In der Figur 3 wird die Hilfsvariable d als ein Schnittmoment $M_X$ in der Höhe x von dem Fußboden bestimmt. In dem dargestellten Beispiel erfolgt die Berechnung auf der Höhe des Fußes, so dass für die Größe x der Wert 0 angenommen werden kann. Das Schnittmoment $M_X$, das auf der Höhe x des unteren Anschlussteils 2 bestimmt wird, errechnet sich nach der Formel

$$d = Mx = M1 + \frac{M2 - M1}{l2 - l1} * (x - l1)$$

wobei $M_1$ das Moment im Verbindungsteil 5 ist, also in der Regel das Knöchelmoment, das Moment $M_2$ das Kniemoment, die Länge $l_1$ die Entfernung des Knöchelmomentsensors zum Fußboden, die Länge $l_2$ die Entfernung des Kniemomentsensors zum Fußboden und die Länge x die Referenzhöhe über dem Boden darstellt, in der das Schnittmoment $M_X$ berechnet werden soll. Hier erfolgt die Berechnung der Hilfsvariable d allein auf der Grundlage der Messung zweier Momentensensoren und der oben beschriebenen mathematischen Verknüpfung. Anhand des Schnittmomentes $M_X$ kann auf die Belastung innerhalb des unteren Anschlussteils 2 geschlossen werden, woraus sich die Belastung innerhalb des unteren Anschlussteils 2 bzw. des Verbindungsteils 5 errechnen lässt. Je nach Größe und Orientierung des Schnittmomentes lassen sich verschiedene Belastungsszenarien erkennen, die eine angepasste Einstellung des Beugeund/oder Streckwiderstandes erfordern. Auf der Grundlage des jeweils momentan wirksamen Schnittmomentes $M_x$, das als Hilfsvariable d in der Steuerung abgelegt ist, kann dann in Echtzeit die jeweils notwendige Verstellung in der Widerstandseinrichtung vorgenommen werden, um den entsprechenden Widerstand einzustellen.

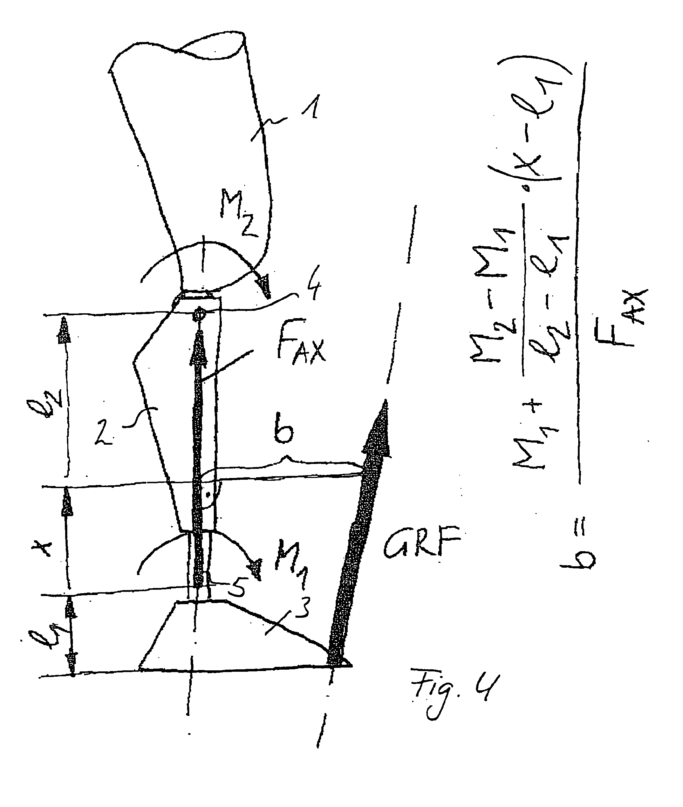

In der Figur 4 ist dargestellt, wie eine weitere Hilfsvariable b in Gestalt des Abstandes des Bodenreaktionskraftvektors GRF zu einer Achse, in diesem Fall die Verbindung der beiden Einrichtungen zum Erfassen von Momenten, in einer Referenzhöhe zu dem Axialkraftvektor $F_{AX}$ berechnet werden kann. Die Hilfsvariable b errechnet sich aus

$$b = \frac{M1 + \frac{M2 - M1}{l2 - l1} * (x - l1)}{FAX}$$

wobei $M_1$ das wirksame Moment im Verbindungsteil 5, zum Beispiel das Knöchelmoment in der Höhe $l_1$ vom Fußboden ist, das Moment $M_2$ das Kniemoment in Höhe der Knieachse 4 ist, die in einem Abstand von $l_2$ zum Fußboden liegt. Die Größe x ist die Referenzhöhe vom Fußboden, die Kraft $F_{AX}$ ist die innerhalb des Verbindungsteils 5 bzw. in dem unteren Anschlussteil 2 wirksame Axialkraft. Über die Veränderung der Hilfsvariable b ist es, wie vorgeschrieben, möglich, kontinuierlich, sowohl während der Schwungphase als auch während der Standphase, die jeweiligen Widerstände einzustellen und an die vorhandenen Veränderungen anzupassen. Dadurch ist es möglich, verschiedene Funktionen zu aktivieren, die automatisch erkannt werden, beispielsweise eine Stehfunktion, über die beispielsweise verhindert wird, dass das Kniegelenk ungewollt einbeugt. Im speziellen Fall wird diese Hilfsvariable auf der Höhe x=0 zum Auslösen der Schwungphase genutzt.

Bei der Bewertung für die Auslösung kann nicht nur das Überschreiten des Schwellwertes für die Hilfsvariable

b$_{(x=0)}$ verwendet werden, sondern auch die Tendenz. So ist bei einem Rückwärtsgehen ein umgekehrter Verlauf der Hilfsvariable anzunehmen, also ein Wandern des Kraftangriffspunktes von der Zehe zu der Ferse. In diesem Fall soll keine Widerstandsverringerung erfolgen.

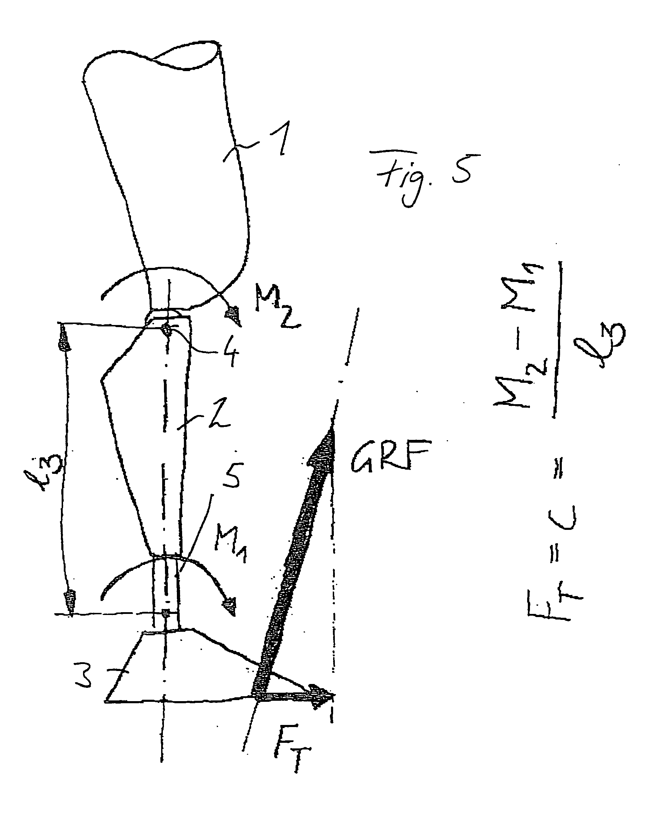

Figur 5 zeigt schematisch, wie die Querkraft oder Tangentialkraft F$_T$ als vierte Hilfsvariable c errechnet und für das Kniesteuerverfahren eingesetzt werden kann. Die Tangentialkraft F$_T$, und damit auch die Hilfsvariable c, ergibt sich aus dem Quotienten der Differenz des Kniemomentes und M$_2$ und dem Knöchelmoment M$_1$ zu dem Abstand l$_3$ zwischen dem Kniemomentsensor und dem Knöchelmomentsensor.

$$c = Ft = \frac{M2 - M1}{l3}$$

Über die Hilfsvariable c kann beispielsweise der Flexionswiderstand in der terminalen Standphase beim Gehen auf schiefen Ebenen mit fallender Hilfsvariable kontinuierlich gesenkt werden, um ein leichteres Durchschwingen des Gelenks zu ermöglichen.

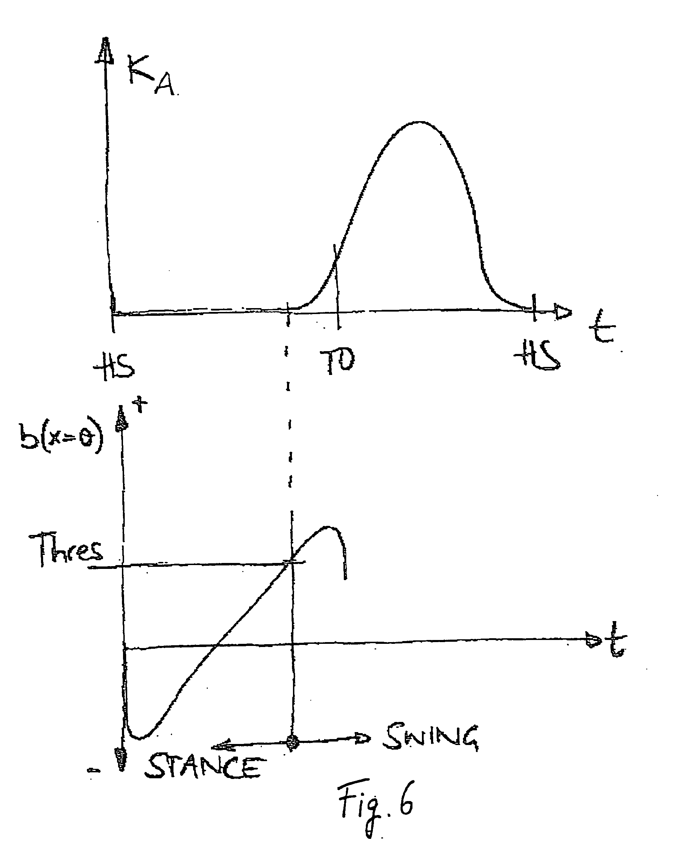

In der Figur 6 ist exemplarisch dargestellt, wie eine Hilfsvariable genutzt werden kann, um das Auslösen der Schwungsphase zu bestimmen. In dem oberen Graph ist der Kniewinkel K$_A$ über die Zeit t aufgetragen, beginnend mit dem Fersenstoß HS und einem im Wesentlichen gleichbleibenden Kniewinkel im Verlauf der Standphase bis zu einem Einbeugen des Knies kurz vor dem Abheben des Vorderfußes zum Zeitpunkt TO. Während der Schwungphase vergrößert sich dann der Kniewinkel K$_A$, bis er nach dem Vorbringen des Fußes bis zum Streckanschlag wieder bei 0 ist und die Ferse erneut aufsetzt.

Unterhalb des Kniewinkeldiagramms ist über die Zeit t der Wert des Abstandes b des Bodenreaktionskraftvektors von der Unterschenkelachse gemäß Figur 4 in Referenzhöhe x=0 aufgezeigt. Sobald die Hilfsvariable b einen Schwellenwert THRES erreicht hat, ist dies für die Steuerung das Auslösesignal, die Widerstände so einzustellen, dass sie für die Schwungphase geeignet sind, beispielsweise durch ein Verringern des Beugewiderstandes zur Erleichterung des Einbeugens kurz bevor der Vorderfuß den Fußboden verlässt. Die Verringerung des Widerstandes kann dabei kontinuierlich erfolgen, nicht schlagartig. Ebenfalls ist es möglich, wenn sich die Hilfsvariable b wieder verändert und einen nicht vorhergesehenen Verlauf nimmt, dass die Widerstände entsprechend angepasst werden, beispielsweise dass der Widerstand erhöht oder das Kniegelenk sogar gesperrt wird.

Neben der dargestellten Steuerung der Funktionen über eine Hilfsvariable ist es möglich, mehrere Hilfsvariable zur Steuerung des künstlichen Gelenkes zu verwenden, um eine präzisere Anpassung an die natürliche Bewegung zu erhalten. Darüber hinaus können weitere Elemente oder Parameter zur Steuerung einer Prothese oder Orthese herangezogen werden, die sich nicht unmittelbar auf die Hilfsvariablen zurückführen lassen.

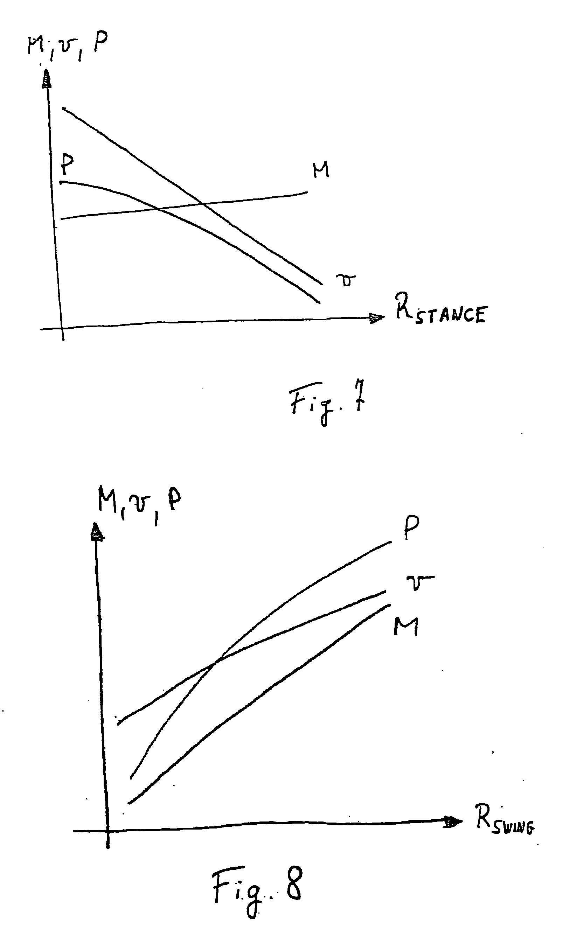

In der Figur 7 ist exemplarisch in dem Diagramm die Abhängigkeit der Kenngrößen Kniemoment M, Leistung P und Geschwindigkeit v über den Widerstand R$_{STANCE}$ in der Standphase bei einem Prothesenkniegelenk aufgetragen. In dem Prothesenkniegelenk sind dabei eine Widerstandseinrichtung und ein Aktuator angeordnet, über den der Widerstand, der der Beugung und/oder Streckung entgegengesetzt wird, verändert werden kann. Neben einer Prothese kann auch eine entsprechend ausgestattete Orthese verwendet werden, ebenfalls sind andere Gelenkeinrichtungen als Einsatzgebiet möglich, beispielsweise Hüft- oder Fußgelenke. In der Widerstandseinrichtung wird in der Regel die mechanische Energie in thermische Energie umgewandelt, um die Bewegung eines Unterschenkelteils relativ zu einem Oberschenkelteil abzubremsen, Entsprechendes gilt für andere Gelenke.

Die Temperatur der Widerstandseinrichtung hängt dabei davon ab, wie groß die Leistung P ist, die während der Standphase aufgebracht wird. Die Leistung P hängt von dem wirksamen Kniemoment M und der Geschwindigkeit v ab, mit der das Kniegelenk gebeugt wird. Diese Geschwindigkeit hängt wiederum von dem Widerstand R$_{STANCE}$ ab, der durch die nicht dargestellte Widerstandseinrichtung der jeweiligen Bewegung in der Standphase entgegengesetzt wird. Werden in der Standphase nach dem Fersenstoß der Flexionswiderstand und im weiteren Verlauf bei einer einsetzenden Extensionsbewegung der Extensionswiderstand erhöht, verringert sich die Bewegungsgeschwindigkeit der Gelenkkomponenten zueinander und damit auch die Bewegungsgeschwindigkeit der Widerstandseinrichtung. Durch die Verringerung der Geschwindigkeit v, die stärker ist als der leichte Anstieg des Momentes M, verringert sich die Leistung P während der Standphase, sodass die umzuwandelnde Energie entsprechend der sich verringernden Leistung P abnimmt. Dementsprechend verringert sich bei einer gleich bleibenden Kühlung die Temperatur der Widerstandseinrichtung oder derjenigen Komponente, die hinsichtlich ihrer Temperatur überwacht wird.

In der Figur 8 ist die Korrelation der beschriebenen Kenngrößen zu dem Widerstand R$_{SWING}$ in der Schwungphase dargestellt. Bei einer Verringerung des Widerstandes R während der Schwungphase verringern sich die Gehgeschwindigkeit v, das Kniemoment M und damit auch die aufgebrachte Leistung P, so dass die umzuwandelnde Energie verringert wird. Dementsprechend verringert sich die Temperatur der Widerstandseinrichtung bei einem abnehmenden Schwungphasenwiderstand. Eine über die Temperatur gesteuerte Stand- und/oder Schwungphasensteuerung kann ergänzend zu der Steuerung über die oben beschriebenen Hilfsvariablen oder auch getrennt davon erfolgen.

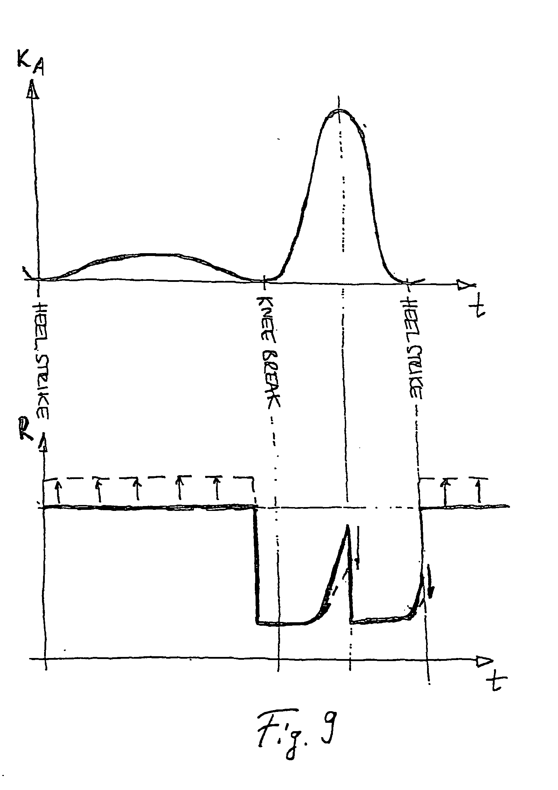

Die Figur 9 zeigt in dem oberen Diagramm den Kniewinkel $K_A$ über die Zeit t, beginnend mit dem so genannten "heel strike", dem Fersenstoß, der in der Regel bei einem gestreckten Kniegelenk ausgeführt wird. Während des Aufsetzens des Fußes erfolgt eine geringe Flexion des Kniegelenkes, die so genannte Standphasenflexion, um das Aufsetzen des Fußes und den Fersenstoß abzumildern. Nachdem der Fuß vollständig aufgesetzt ist, ist das Kniegelenk vollständig gestreckt, bis zum so genannten "knee break", bei dem das Kniegelenk eingeknickt wird, um das Kniegelenk nach vorne zu bewegen und über den Vorderfuß abzurollen. Von dem "knee break" ausgehend erhöht sich der Kniewinkel $K_A$ bis zum maximalen Kniewinkel in der Schwungphase, um dann wieder nach dem Vorbringen des gebeugten Beines und des Prothesenfußes in eine Streckstellung überzugehen, um dann wieder mit der Ferse aufzusetzen. Dieser Kniewinkelverlauf ist typisch für das Gehen in der Ebene.

In der unteren Grafik ist der Widerstand R über die Zeit aufgetragen, korrespondierend zu dem entsprechenden Kniewinkel. Aus dieser Graphik wird ersichtlich, wie sich eine Veränderung des Widerstandes in der Schwungphase und der Standphase auswirkt, die beispielsweise aufgrund einer temperaturinduzierten Widerstandsveränderung durchgeführt wurde. Ob ein Extensions- oder Flexionswiderstand vorliegt, hängt von dem Kniewinkelverlauf ab, bei zunehmendem Kniewinkel $K_A$ ist der Flexionswiderstand wirksam, bei abnehmendem Kniewinkel der Extensionswiderstand. Nach dem "heel strike" ist ein relativ hoher Flexionswiderstand vorhanden, nach der Bewegungsumkehr ein hoher Extensionswiderstand. Bei "knee break" wird der Widerstand reduziert wird, um das Einbeugen und Vorbringen des Knies zu erleichtern. Dadurch wird das Gehen erleichtert. Nach dem Absenken des Widerstandes beim "knee break" wird der Widerstand über einen Teil der Schwungphase auf dem niedrigen Niveau gehalten, um ein nach hinten Schwingen des Prothesenfußes zu erleichtern. Um die Schwungbewegung nicht zu groß werden zu lassen, wird vor Erreichen des Kniewinkelmaximums der Flexionswiderstand erhöht und nach Erreichen des Kniewinkelmaximums und der Bewegungsumkehr der Extensionswiderstand auf das niedrige Niveau der Schwungphasenbeugung verringert. Die Widerstandsverringerung des Extensionswiderstandes bleibt auch über die Extensionsbewegung in der Schwungphase erhalten, bis kurz vor dem "heel strike". Kurz vor Erreichen der vollständigen Streckung wird der Widerstand erneut erhöht, um ein hartes Anschlagen in den Streckanschlag zu vermeiden. Um beim Aufsetzen des Prothesenfußes eine ausreichende Sicherheit gegen ein unkontrolliertes Einknicken zu erhalten, ist auch der Flexionswiderstand auf einem hohen Niveau.

Wird nun der Flexionswiderstand erhöht, was durch die gestrichelte Linie angedeutet ist, verlangsamt sich die Kniewinkelgeschwindigkeit und damit auch das Gehen des Prothesennutzers. Nach dem "heel strike" folgt nur eine vergleichsweise geringe Beugung in der Standphasenflexion und eine langsame Streckung, so dass weniger Energie dissipiert wird. Das Anheben des Flexionswiderstandes vor Erreichen des Kniewinkelmaximums erfolgt in einer weniger ausgeprägten Weise als bei der Standard-Dämpfung, was durch den nach unten gerichteten Pfeil angedeutet ist. Dadurch schwingt der Unterschenkel und damit der Prothesenfuß weiter aus, so dass ein größerer Zeitraum zwischen den "heel strikes" vorliegt. Auch das Verringern des Flexionswiderstandes in der Schwungphasenflexion führt zu einer Verringerung der Gehgeschwindigkeit.

Am Ende der Schwungphasenextension, also kurz vor dem Auftreten und dem "heel strike", wird der Extensionswiderstand im Vergleich zu dem Standard-Niveau verringert. Es ist also vorgesehen, dass der Extensionswiderstand verringert wird, so dass das Unterschenkelteil schneller in die Streckung gelangt. Um ein hartes Anschlagen in die Streckung zu vermeiden, wird der Prothesennutzer langsamer gehen, so dass sich die Leistung P und damit die zu dissipierende Energie verringert. Während der Standphase zwischen dem "heel strike" und dem "knee break" können sowohl der Flexionswiderstand als auch der Extensionswiderstand erhöht werden, um die leichte Beuge- und Streckbewegung zu verlangsamen, um dadurch die Gehgeschwindigkeit zu verringern.

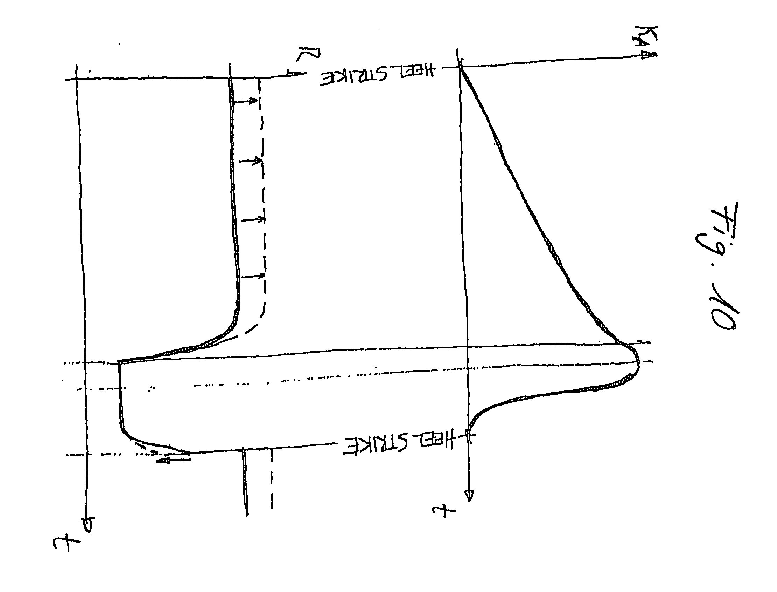

In der Figur 10 ist in der oberen Darstellung der Kniewinkelverlauf beim Gehen auf einer Rampe gezeigt, hier auf einer abschüssigen Rampe. Nach dem "heel strike" erfolgt eine kontinuierliche Vergrößerung des Kniewinkels $K_A$ bis zum Kniewinkelmaximum, ohne dass ein "knee break" stattfindet. Dies liegt darin begründet, dass beim Gehen auf einer Rampe das Knie nicht in eine vollständige Streckung gelangt. Nach dem Erreichen des Kniewinkelmaximums erfolgt ein schnelles Vorbringen des Knies und des Unterschenkels bis zu einer vollständigen Streckung, die mit dem erneuten "heel strike" einhergeht. Der Flexionswiderstand bleibt dabei über einen weiten Verlauf auf einem konstant hohen Niveau, bis er dann abgesenkt wird, um ein weites Einbeugen des Knies und damit ein Abheben des Prothesenfußes und ein Durchschwingen zu ermöglichen. Dieses Durchschwingen findet nach Erreichen des Minimums des Widerstandes bis zum Erreichen des Kniewinkelmaximums statt. Anschließend wird der Extensionswiderstand auf einem niedrigen Niveau gehalten, bis er kurz vor dem Auftreten erneut angehoben wird.

Liegen nun erhöhte Temperaturen in der Widerstandseinrichtung vor, werden die Widerstände in der Standphase erhöht, um eine langsame Gehgeschwindigkeit und ein langsames Einknicken zu gewährleisten. Nach dem Erreichen des maximalen Beugewinkels in der Schwungphase wird beim nach vorne Bringen des Prothesenfußes der Extensionswiderstand im Vergleich zur Normalfunktion verringert, was ebenfalls zu einer Verringerung der zu dissipierenden Energie führt.

Neben der üblichen Bewegungssituation, in der ein Patient sich vorwärts bewegt, sind in dem täglichen Bewegungsprofil viele andere Situationen vorhanden, auf die mit einer angepassten Steuerung reagiert werden sollte.

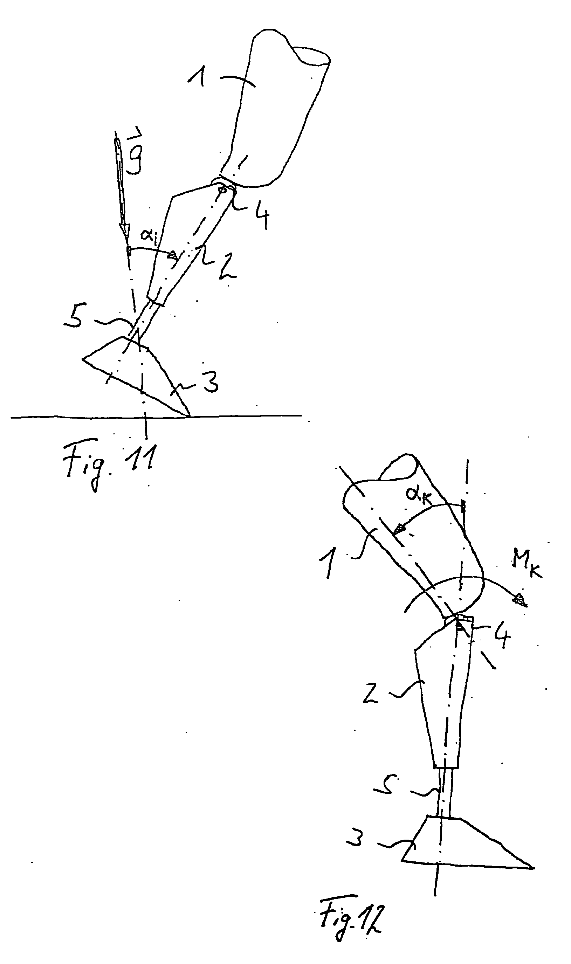

In der Figur 11 ist die Prothese in einer Situation dargestellt, in der normalerweise beim Vorwärtsgehen die Schwungphase ausgelöst wird. Der Patient steht in dieser Situation noch auf dem Vorderfuß und möchte dann die Hüfte beugen, so dass auch das Knie einbeugt. In die gleiche Situation kommt der Patient aber auch beim Rückwärtsgehen. Ausgehend von einer stehenden Situation wird beim Rückwärtsgehen das versorgte Bein, vorliegend die Prothese, nach hinten gesetzt, also der normalen Blickrichtung eines Prothesennutzers entgegengesetzt. Dadurch ergibt sich, dass der Inertialwinkel $\alpha_1$ des Unterschenkelteils 2 zu der Schwerkraftrichtung, die durch den Schwerkraftvektor g angedeutet ist, zunächst zunimmt, bis der Prothesenfuß 3 auf den Boden aufgesetzt wird. Als Drehpunkt oder Angelpunkt für die Bewegung und zur Bestimmung des zunehmenden Inertialwinkels $\alpha_1$ ist dabei das Hüftgelenk anzunehmen. Die Längserstreckung oder Längsachse des Unterschenkelteils 2 verläuft durch die Schwenkachse des Prothesenkniegelenkes 4 und bevorzugt ebenfalls durch eine Schwenkachse des Knöchelgelenkes oder aber zentral durch eine Anschlussstelle zwischen dem Prothesenfuß 3 und dem Unterschenkelteil 2. Der Inertialwinkel $\alpha_1$ des Unterschenkelteils 2 kann direkt durch eine an dem Unterschenkelteil 2 angeordnete Sensorik bestimmt werden, alternativ dazu kann dieser über eine Sensorik an dem Oberschenkelteil 1 und einem Kniewinkelsensor bestimmt werden, der den Winkel zwischen dem Oberschenkelteil 1 und dem Unterschenkelteil 2 erfasst.

Zur Bestimmung der Inertialwinkelgeschwindigkeit kann direkt ein Gyroskop verwendet werden oder es wird die Veränderung des Inertialwinkels $\alpha_1$ über die Zeit ermittelt, die mit Betrag und Richtung ermittelt werden kann. Liegt nun ein bestimmter Inertialwinkel $\alpha_1$ und eine bestimmte Inertialwinkelgeschwindigkeit $\omega_1$ vor, wird eine Schwungphase eingeleitet, falls ein bestimmter Schwellenwert für die Inertialwinkelgeschwindigkeit $\omega_1$ überschritten wird. Liegt ein abnehmender Inertialwinkel $\alpha_1$ vor und darüber hinaus noch eine Vorfußbelastung, kann auf ein Rückwärtsgehen geschlossen werden, so dass der Flexionswiderstand nicht verringert, sondern beibehalten oder erhöht wird, um keine Schwungphasenflexion einzuleiten.

In der Figur 12 ist die Prothese in einem flach auf dem Boden aufgesetzten Zustand gezeigt. Die Darstellung dient insbesondere zur Definition des Kniemomentes und des Kniewinkels sowie der verwendeten Vorzeichenkonvention. Der Kniewinkel $\alpha_K$ entspricht dabei dem Winkel zwischen dem Oberschenkelteil 1 und dem Unterteil 2. Um die Gelenkachse des Prothesenkniegelenkes 4 ist ein Kniemoment $M_K$ wirksam. Die Auslösung der Schwungphase kann durch weitere Kriterien ergänzt werden, beispielsweise dadurch, dass das Kniemoment $M_K$ streckend sein muss, also positiv oder null, dass der Kniewinkel $\alpha_K$ nahezu Null ist, also dass das Knie gestreckt ist und/oder dass die Kniewinkelgeschwindigkeit null oder streckend ist.

Eine elegante Art und Weise, verschiedene Parameter und Parameterzusammenhänge zu berücksichtigen, ist durch den Einsatz eines Kennfeldes gegeben. Das Kennfeld ermöglicht es, anders als eine rein über einen Schwellwert gesteuerte Schaltung, variable und an Verläufe oder Kombinationen der Kennfeldgrößen angepasste Widerstände einzustellen. Dabei können auch die Hilfsvariablen verwendet werden, die oben beschrieben wurden.

In Figur 13 ist ein Kennfeld zur Steuerung für das Gehen in der Ebene dargestellt, das zur Ermittlung des einzustellenden Widerstandes R aufgestellt wurde. Das Kennfeld ist zwischen dem Widerstand R, dem Kniewinkel $K_A$ sowie dem Kniehebel $K_L$ aufgespannt. Der Kniehebel $K_L$ ist der Normalabstand der resultierenden Bodenreaktionskraft zur Knieachse und kann durch die Division des wirksamen Kniemomentes durch die wirksame Axialkraft errechnet werden, wie es in der Figur 2 beschrieben wurde. Dort wurde der Kniehebel als Hilfsvariable a -allerdings mit umgekehrtem Vorzeichen- beschrieben. Als Maximalwert für den Widerstand R wird diejenige Größe angenommen, bei der ohne Zerstörung einer Komponente das Gelenk, vorliegend das Kniegelenk, nicht oder nur sehr langsam gebeugt werden kann. Wenn der Kniehebel $K_L$ = -a nach einer anfänglichen Zunahme gegen Null strebt und der Unterschenkel deutlich nach hinten gekippt war, was typisch für das Gehen in der Ebene ist, wird der Flexionswiderstand R ausgehend von einem Basisflexionswiderstand bis zu einem maximalen Standphasenbeugewinkel von beispielsweise 15° oder knapp darunter mit zunehmenden Kniewinkel bis zum Blockwiderstand $R_{BLOCK}$ erhöht. Eine solche Kurve ist als normale Standphasenflexionskurve $R_{SF}$ in der Figur 13 dargestellt. Die Widerstandseinrichtung begrenzt also das Einbeugen bei Standphasenflexion beim Gehen in der Ebene. Wenn der Kniehebel $K_L$ jedoch zunimmt, wird der Flexionswidersand weniger erhöht. Dieses Verhalten entspricht z.B. dem Abwärtsgehen auf einer Rampe oder einem Bremsschritt und ist mit $R_{RAMP}$ eingezeichnet. Durch das Kennfeld wird ein kontinuierlicher Übergang zwischen dem Gehen in der Ebene und dem Gehen auf einer Rampe möglich. Nachdem kein Schwellwert, sondern ein kontinuierliches Kennfeld verwendet wird, ist auch im fortgeschrittenen Stadium der Standphase ein Übergang zwischen Gehen in der Ebene und Rampengehen möglich.

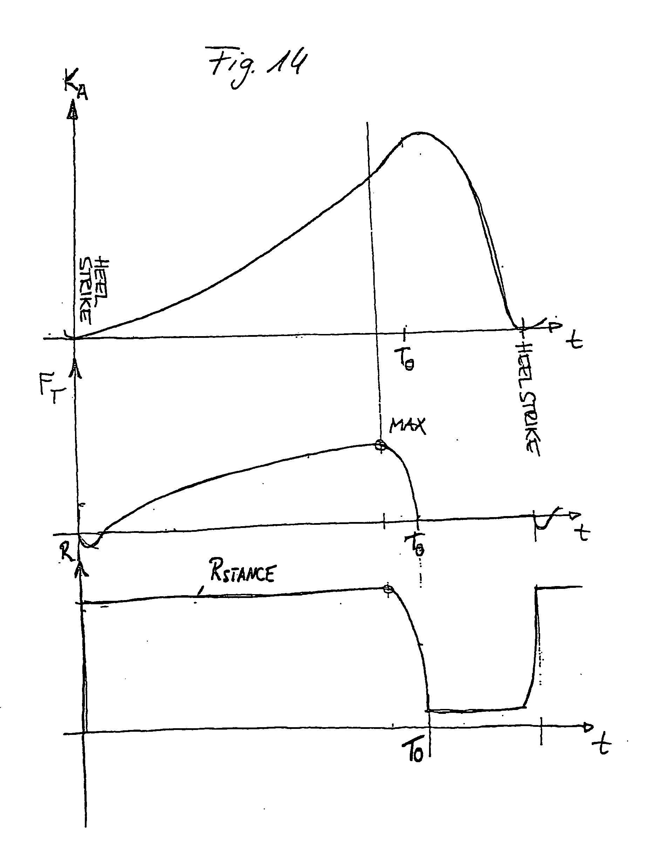

In der Figur 14 sind die charakteristischen Kenngrößen Kniewinkel $K_A$, Tangentialkraft $F_T$ sowie Flexionswiderstand R über die Zeit t beim Gehen auf schiefen Ebenen dargestellt, vorliegend beim Bergabgehen. Nach dem "heel strike" erhöht sich kontinuierlich der Kniewinkel $K_A$ bis zum Zeitpunkt des Abhebens des Fußes $T_O$. Danach erhöht sich der Kniewinkel $K_A$ noch einmal, um in der Schwungphase das Unterschenkelteil näher an das Oberschenkelteil heranzubringen, um den Fuß nach vorne setzen zu können. Nach Erreichen des maximalen Kniewinkels $K_A$ wird das Unterschenkelteil nach vorne gebracht, der Kniewinkel $K_A$ verringert sich bis auf Null, sodass sich das Bein wieder im gestreckten Zustand befindet, in dem die Ferse aufgesetzt wird, sodass ein neuer Schrittzyklus beginnen kann.

Die Tangentialkraft $F_T$ oder Querkraft nimmt nach dem "heel strike" einen negativen Wert an, erfährt einen Nulldurchgang nach dem vollständigen Aufsetzen des Fußes und steigt dann auf einen Maximalwert kurz vor dem Abheben des Fußes an. Nach dem Abheben des Fußes zum Zeitpunkt $T_0$ ist die Querkraft $F_T$ Null, bis zum erneuten "heel strike".

Der Verlauf des Flexionswiderstandes R ist bis zum Maximum der Querkraft $F_T$ nahezu konstant und sehr hoch, um der beim Bergabgehen in Flexionsrichtung wirkenden Kraft entgegen zu wirken, damit der Patient entlastet wird und nicht durch die erhaltene Seite den Schwung des bewegten künstlichen Knies kompensieren muss. Nach Erreichen des Querkraftmaximums, das vor dem Abheben des Fußes liegt, wird der Flexionswiderstand R kontinuierlich mit der Tangentialkraft verringert, um ein erleichtertes Einbeugen des Kniegelenkes zu ermöglichen. Nach dem Abheben des Vorderfußes zum Zeitpunkt $T_0$ hat der Flexionswiderstand R seinen Minimalwert, damit der Unterschenkel wieder leicht nach hinten schwingen kann. Wird der Unterschenkel nach vorne gebracht, ist der Extensionswiderstand wirksam, der aus Gründen der Vollständigkeit in dieses Diagramm mit eingezeichnet ist. Bei einem abnehmenden Kniewinkel ist der Widerstand R als Extensionswiderstand ausgebildet, der kurz vor Erreichen des erneuten Aufsetzens, also kurz vor dem erneuten "heel strike", auf einen maximalen Wert erhöht wird, um eine Extensionsdämpfung bereitzustellen, damit das Kniegelenk nicht in den Extensionsanschlag ungedämpft hineinbewegt wird. Der Flexionswiderstand wird auf den hohen Wert erhöht, damit unmittelbar nach dem "heel strike" der benötigte wirksame Flexionswiderstand bereitgestellt werden kann.

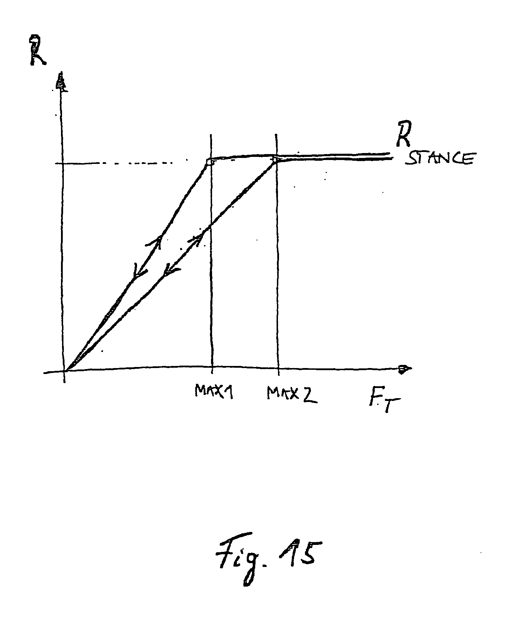

In der Figur 15 ist das Verhältnis zwischen dem einzustellenden Widerstand R und unterschiedlichen Querkraftmaxima dargestellt. Die Widerstandabnahme ist dabei auf das Querkraftmaximum normiert. Damit soll erreicht werden, dass der Widerstand von einen hohem Wert auf einen niedrigen Wert heruntergefahren wird, während die Querkraft von einem Maximum auf den Wert null geht. Die Reduzierung ist damit von der Höhe des Maximums der Querkraft unabhängig. Sie geht von dem Standphasenwiderstand bis zum Minimalwiderstand, während die Querkraft vom Maximum zu Null geht. Sollte die Querkraft wieder steigen, wird der Widerstand wieder erhöht, dass heißt, der Prothesennutzer kann das Gelenk wieder stärker belasten, sollte er die Bewegung abbrechen. Auch hier ist ein kontinuierlicher Übergang zwischen einem leichten Durchschwingen und einer Wiederbelastung möglich, ohne dass ein diskretes Schaltkriterium verwendet wird.

**Patentansprüche**

1. Verfahren zur Steuerung eines orthetischen oder prothetischen Gelenkes (4) einer unteren Extremität mit einer Widerstandseinrichtung, der zumindest ein Aktuator zugeordnet ist, über den der Beuge- und/oder Streckwiderstand (R) in Abhängigkeit von Sensordaten verändert wird, wobei über Sensoren während der Benutzung des Gelenkes Zustandsinformationen bereitgestellt werden, wobei die Sensordaten von zumindest einer Einrichtung zur Erfassung von zumindest

   - einem Moment und einer Kraft, oder
   - zwei Momenten und einer Kraft, oder
   - zwei Kräften und einem Moment

   ermittelt werden und die Sensordaten von zumindest zwei der ermittelten Größen durch eine mathematische Operation miteinander verknüpft werden und dadurch *zumindest* eine Hilfsvariable (a, b) errechnet wird, die der Steuerung des Beuge- und/oder Streckwiderstandes (R) zugrundegelegt wird, **dadurch gekennzeichnet, dass** als Hilfsvariable (a, b) der Abstand des Kraftvektors der Bodenreaktionskraft (GRF) von der Einrichtung zur Erfassung eines Momentes durch die Division des Momentes (M) durch die Kraft (GRF) errechnet wird.

2. Verfahren nach Anspruch 1, **dadurch gekennzeichnet, dass** der Abstand des Kraftvektors zur Gelenkachse durch die Division des Gelenkmomentes durch die Axialkraft (FAX) errechnet wird.

3. Verfahren nach Anspruch 1 oder 2, **dadurch gekennzeichnet, dass** als Hilfsvariable (a, b) der Abstand des Kraftvektors zu einer Achse eines Gelenkanschlussteiles in einer Referenzposition durch Verknüpfung der Daten von zumindest einer Einrichtung zur Erfassung zweier Momente und einer Kraft ermittelt wird.

4. Verfahren nach einem der voranstehenden Ansprüche, **dadurch gekennzeichnet, dass** als Hilfsvariable ein Schnittmoment in einer Referenzhöhe durch gewichtete Addition oder Subtraktion der Werte von Einrichtungen zur Erfassung zweier Momente, insbesondere eines Knöchelmomentsensors und eines Kniemomentsensors, ermittelt wird.

**5.** Verfahren nach einem der voranstehenden Ansprüche, **dadurch gekennzeichnet, dass** als Hilfsvariable (c) eine auf ein unteres Anschlussteil (2) ausgeübte Querkraft (Ft) aus dem Quotienten der Differenz zweier Momente (M1, M2) und dem Abstand (l3) der beiden Einrichtungen zur Ermittlung der Momente zueinander ermittelt wird.

**6.** Verfahren nach einem der voranstehenden Ansprüche, **dadurch gekennzeichnet, dass** bei Erreichen oder Überschreiten eines vorbestimmten Wertes für die Hilfsvariable die Widerstandseinrichtung in einen Schwungphasenzustand geschaltet wird.

**7.** Verfahren nach einem der voranstehenden Ansprüche, **dadurch gekennzeichnet, dass** bei einer festgestellten Verringerung der Bodenreaktionskraft (GRF) auf die Orthese oder Prothese der Widerstand (R) verringert und bei einer zunehmenden Bodenreaktionskraft (GRF) der Widerstand (R) bis zu einer Sperrung des Gelenkes erhöht wird.

**8.** Verfahren nach Anspruch 7, **dadurch gekennzeichnet, dass** die Sperrung des Gelenkes (4) aufgehoben wird, wenn die Hilfsvariable sich ändert.

**9.** Verfahren nach einem der voranstehenden Ansprüche, **dadurch gekennzeichnet, dass** der Widerstand (R) nach der Erhöhung auf der Grundlage einer erfassten Veränderung der Lage der Orthese oder Prothese im Raum oder durch eine erfasste Veränderung der Lage eines Kraftvektors zu der Orthese oder Prothese verringert wird.

**10.** Verfahren nach einem der voranstehenden Ansprüche, **dadurch gekennzeichnet, dass** ein Temperatursensor vorgesehen ist und dass der Widerstand (R) in Abhängigkeit von zumindest einem gemessenen Temperatursignal verändert wird, wobei die Temperatur der Widerstandseinrichtung gemessen und der Steuerung zu Grunde gelegt wird.

**11.** Verfahren nach Anspruch 10, **dadurch gekennzeichnet, dass** während der Standphase bei zunehmender Temperatur der Widerstand (R) erhöht oder dass während der Schwungphase der Beugewiderstand (R) bei zunehmender Temperatur verringert wird.

**12.** Verfahren nach einem der voranstehenden Ansprüche, **dadurch gekennzeichnet, dass** im Falle eines Ausfalles von Einrichtungen zur Erfassung von Momenten (M1, M2), Kräften (Ft, FAX) und/oder Gelenkwinkeln ($K_A$) alternative Steueralgorithmen auf Basis der restlichen Einrichtungen zur Veränderung des Streck- und/oder Beugewiderstandes (R) verwendet werden.

**13.** Verfahren nach einem der voranstehenden Ansprüche, **dadurch gekennzeichnet, dass** der Abstand (a, b) des Bodenreaktionskraftvektors (GRF) zu einem Gelenkteil ermittelt wird und der Widerstand (R) reduziert wird, wenn ein Schwellwert des Abstandes (a, b) überschritten wird.

**14.** Verfahren nach Anspruch 13, **dadurch gekennzeichnet, dass** der Widerstand (R) nach einer Reduzierung wieder auf den Wert für die Standphase erhöht wird, wenn innerhalb einer festgelegten Zeit nach der Reduzierung des Widerstandes (R) ein Schwellwert für einen Inertialwinkel ($\alpha_I$) einer Gelenkkomponente, für eine Inertialwinkelgeschwindigkeit ($\omega_I$), für eine Bodenreaktionskraft (GRF), für ein Gelenkmoment (M), für einen Gelenkwinkel ($K_A$) oder für einen Abstand (a, b) eines Kraftvektors (GRF, FAX) zu einer Gelenkkomponente nicht erreicht wird.

**15.** Verfahren nach einem der voranstehenden Ansprüche, **dadurch gekennzeichnet, dass** der Kraftangriffspunkt an einem Fuß (3) bestimmt wird und der Widerstand (R) erhöht oder nicht verringert wird, wenn sich der Kraftangriffspunkt in Richtung Ferse bewegt.

**16.** Verfahren nach einem der voranstehenden Ansprüche, **dadurch gekennzeichnet, dass** der Beugewiderstand in der Standphase erhöht oder nicht verringert wird, wenn ein in Richtung auf die Vertikale abnehmender Inertialwinkel eines Unterschenkelteils und gleichzeitig ein belasteter Vorfuß ermittelt werden.

**Claims**

**1.** A method for controlling an orthotic or prosthetic joint (4) of a lower extremity with a resistance device, which is assigned at least one actuator by way of which the bending and/or stretching resistance is changed in dependence on sensor data, information pertaining to the state being provided by way of sensors during the use of the joint, wherein the sensor data are determined by at least one device for detecting at least

- one torque and one force, or
- two torques and one force, or
- two forces and one torque

and the sensor data of at least two of the variables determined are linked to one another by a mathematical operation and, as a result, at least one auxiliary variable (a, b) is calculated and used as a basis for controlling the bending and/or stretching resistance (R), **characterized in that** the distance of the force vector of the ground reaction force (GRF) from the device for detecting a torque is calculated as the auxiliary variable (a, b) by dividing the torque (M) by the force (GRF).

2. The method as claimed in claim 1, **characterized in that** the distance of the force vector from the joint axis is calculated by dividing the joint torque by the axial forces (FAX).

3. The method as claimed in claim 1 or 2, **characterized in that** the distance of the force vector from an axis of a joint connection part in a reference position is determined as the auxiliary variable (a, b) by linking the data of at least one device for detecting two torques and one force.

4. The method as claimed in one of the preceding claims, **characterized in that** a sectional torque at a reference height is determined as the auxiliary variable by weighted addition or subtraction of the values of devices for detecting two torques, in particular an ankle torque sensor and a knee torque sensor.

5. The method as claimed in one of the preceding claims, **characterized in that** a transverse force (Ft) exerted on a lower connection part (2) is determined as auxiliary variable (c) from the quotient of the difference between two torques (M1, M2) and the distance (13) between two torques and the distance between the two devices for determining the torques.

6. The method as claimed in one of the preceding claims, **characterized in that**, when a predetermined value for the auxiliary variable is reached or exceeded, the resistance device is switched into a swing phase state.

7. The method as claimed in one of the preceding claims, **characterized in that**, when there is an established reduction of the ground reaction force (GRF) on the orthesis or prosthesis, the resistance (R) is reduced and, when there is an increasing ground reaction force (GRF), the resistance (R) is increased up to a locking of the joint.

8. The method as claimed in claim 7, **characterized in that** the locking of the joint is canceled if the auxiliary variable changes.

9. The method as claimed in one of the preceding claims, **characterized in that** the resistance (R) is reduced after the increase on the basis of a detected changing of the spatial position of the orthesis or prosthesis or as a result of a detected changing of the position of a force vector in relation to the orthesis or prosthesis.

10. The method is claimed in one of the preceding claims, **characterized in that** a temperature sensor is provided and **in that** the resistance (R) is changed in dependence on at least one measured temperature signal, whereby the temperature of the resistance device is measured and used as a basis for the control.

11. The method as claimed in claim 10, **characterized in that** the resistance (R) is increased during the standing phase when there is increasing temperature or the bending resistance is reduced during the swing phase when there is increasing temperature.

12. The method as claimed in one of the preceding claims, **characterized in that**, in the case of a failure of devices for detecting torques (M1, M2), forces (Ft, FAX) and/or joint angles (K$_A$), alternative control algorithms on the basis of the remaining devices are used for changing the stretching and/or bending resistance (R).

13. The method as claimed in one of the preceding claims, **characterized in that** the distance (a, b) of the ground reaction force (GRF) vector from a joint part is determined and the resistance (R) is reduced if a threshold value of the distance (a, b) is exceeded.

14. The method as claimed in claim 13, **characterized in that**, after a reduction, the resistance (R) is increased again to the value for the standing phase if, within a fixed time after the reduction of the resistance (R), a threshold value for an inertial angle ($\alpha_I$) of a joint component, for an inertial angle velocity ($\dot{\omega}_I$), for a ground reaction force (GRF), for a joint torque (M), for a joint angle ($K_A$) or for a distance (a, b) of a force vector (GRF, FAX) from a joint component is not reached.

15. The method as claimed in one of the preceding claims, **characterized in that** the point at which a force acts on the foot (3) is determined and the resistance (R) is increased, or not reduced, if the point at which a force acts moves in the direction to the heel.

16. The method as claimed in one of the preceding claims, **characterized in that** the bending resistance is increased, or not reduced, in the standing phase if an inertial angle of a lower leg part that is decreasing in the direction of the vertical and simultaneously a loading of the forefoot are determined.

**Revendications**

1. Procédé pour la commande d'une articulation orthétique ou prothétique (4) d'une extrémité inférieure comprenant un système de résistance auquel est associé au moins un actionneur au moyen duquel la résistance à la flexion et/ou à l'extension (R) est modifiée en fonction de données sensorielles, dans lequel on prépare pendant l'utilisation de l'articulation des informations d'état au moyen de capteurs, lesdites données sensorielles étant déterminées par au moins un système pour détecter au moins

   - un couple et une force, ou
   - deux couples et une force, ou
   - deux forces et un couple

   et les données sensorielles d'au moins deux des grandeurs déterminées sont chaînées les unes aux autres par une opération mathématique et l'on calcule grâce à cela au moins une variable auxiliaire (a, b), qui est mise à la base de la commande de la résistance à la flexion et/ou à l'extension (R), **caractérisé en ce que** l'on calcule à titre de variable auxiliaire (a, b) la distance du vecteur-force de la force de réaction au sol (GRF) du système pour la détection d'un couple par la division du couple (M) par la force (GRF).

2. Procédé selon la revendication 1, **caractérisé en ce que** la distance du vecteur-force à l'axe de l'articulation est calculée par la division du couple d'articulation par la force axiale (FAX).

3. Procédé selon la revendication 1 ou 2, **caractérisé en ce que** l'on détermine à titre de variable auxiliaire (a, b) la distance du vecteur-force à un axe d'une partie de raccordement d'articulation dans une position de référence par chaînage des données d'au moins un système pour la détection de deux couples et d'une force.

4. Procédé selon l'une des revendications précédentes, **caractérisé en ce que** l'on détermine à titre de variable auxiliaire un couple d'intersection à une hauteur de référence par addition ou soustraction pondérée des valeurs de systèmes pour la détection de deux couples, en particulier d'un détecteur de couple de cheville et d'un détecteur de couple de genou.

5. Procédé selon l'une des revendications précédentes, **caractérisé en ce que** l'on détermine à titre de variable auxiliaire (c) une force transversale (Ft) exercée sur une partie de raccordement inférieure (2) à partir du quotient de la différence de deux couples ($M_1$, $M_2$) et de la distance (l3) des deux systèmes pour la détermination des couples l'un par rapport à l'autre.

6. Procédé selon l'une des revendications précédentes, **caractérisé en ce que** lorsque l'on atteint ou on dépasse une valeur prédéterminée pour la variable auxiliaire le système de résistance est commuté dans un état de phase d'élan.

7. Procédé selon l'une des revendications précédentes, **caractérisé en ce que** lors de la constatation d'une réduction de la force de réaction au sol (GRF) sur l'orthèse ou la prothèse, la résistance (R) est réduite et dans le cas d'une augmentation de la force de réaction au sol (GRF) la résistance (R) est augmentée jusqu'à un blocage de l'articulation.

8. Procédé selon la revendication 7, **caractérisé en ce que** le blocage de l'articulation (4) est annulé quand la variable auxiliaire est modifiée.

9.  Procédé selon l'une des revendications précédentes, **caractérisé en ce que**, après l'augmentation, la résistance est réduite sur la base de la détection d'une modification de la situation de l'orthèse ou de la prothèse dans l'espace ou par la détection d'une modification de la position d'un vecteur-force par rapport à l'orthèse ou à la prothèse.

10. Procédé selon l'une des revendications précédentes, **caractérisé en ce qu'**il est prévu un capteur de température, et **en ce que** la résistance (R) est modifiée en fonction d'au moins un signal de température mesurée, dans lequel la température du système de résistance est mesurée et est mise à la base de la commande.

11. Procédé selon la revendication 10, **caractérisé en ce que** pendant la phase en station debout la résistance (R) est augmentée lorsque la température augmente, ou **en ce que** pendant la phase d'élan, la résistance à la flexion (R) est réduite lorsque la température augmente.

12. Procédé selon l'une des revendications précédentes, **caractérisé en ce que** dans le cas d'une défection de systèmes pour la détection de couples ($M_1$, $M_2$), de forces (Ft, FAX) et/ou d'angles d'articulation ($K_A$), on utilise des algorithmes de commandes alternatifs en se basant sur les systèmes restants pour la modification de la résistance à l'extension et/ou à la flexion (R).

13. Procédé selon l'une des revendications précédentes, **caractérisé en ce que** la distance (a, b) du vecteur de la force de réaction au sol (GRF) jusqu'à une partie de l'articulation est déterminée, et la résistance (R) est réduite lorsqu'on dépasse une valeur seuil de la distance (a, b).

14. Procédé selon la revendication 13, **caractérisé en ce que**, après une réduction, la résistance (R) est à nouveau augmentée à la valeur pour la phase en station debout si à l'intérieur d'un temps fixé après la réduction de la résistance (R), une valeur seuil pour un angle d'inertie ($\alpha_1$) d'un composant de l'articulation, pour une vitesse de l'angle d'inertie ($\omega_1$), pour une force de réaction au sol (GRF), pour un couple d'articulation (M), pour un angle d'articulation ($K_A$) ou pour une distance (a, b) d'un vecteur-force (GRF, FAX) jusqu'à un composant de l'articulation n'est pas atteinte.

15. Procédé selon l'une des revendications précédentes, **caractérisé en ce que** l'on détermine le point d'attaque de force au niveau d'un pied (3) et l'on augmente la résistance (R) ou on ne la réduit pas si le point d'attaque de force se déplace en direction du talon.

16. Procédé selon l'une des revendications précédentes, **caractérisé en ce que** la résistance à la flexion est augmentée dans la phase en station debout ou n'est pas réduite si l'on détermine qu'un angle d'inertie d'une partie de jambe diminue en direction de la verticale, et que l'on détermine simultanément que la partie antérieure du pied est chargée.

Fig.1

$$a = \frac{M}{F_{AX}}$$

Fig. 2

Fig. 3

$$d = M_x = M_1 + \frac{M_2 - M_1}{\ell_2 - \ell_1} \cdot (x - \ell_1)$$

EP 2 498 727 B1

Fig. 4

$$b = \cfrac{M_1 + \cfrac{M_2 - M_1}{\ell_2 - \ell_1} \cdot (x - \ell_1)}{F_{AX}}$$

Fig. 5

$$F_T = C = \frac{M_2 - M_1}{\ell_3}$$

Fig. 6

Fig. 7

Fig. 8

Fig. 9

Fig. 10

Fig. 11

Fig.12

Fig. 13

Fig. 14

Fig. 15

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008008284 A1
- DE 102006021802 A1
- WO 0143669 A1
- US 20080228287 A1
- US 20090030530 A1
- US 2008114272 A1
- WO 9641599 A1